# EUROPEAN PATENT SPECIFICATION

(11) **EP 3 852 896 B1**
(45) Date of publication and mention of the grant of the patent: **06.05.2026**
(21) Application number: 19779727.7
(22) Date of filing: 19.09.2019
(51) Int. Cl.: B01D 15/30, G01N 30/88, G01N 30/72, G01N 33/92, G01N 30/04

(54) **SYSTEM AND METHOD FOR LIPID QUANTIFICATION**
SYSTEM UND VERFAHREN ZUR LIPIDQUANTIFIZIERUNG
SYSTÈME ET PROCÉDÉ POUR LA QUANTIFICATION DE LIPIDES

(30) Priority: 21.09.2018 US 201862734410 P
(43) Date of publication of application: 28.07.2021
(73) Proprietor: Waters Technologies Corporation, Milford, MA 01757 (US)
(72) Inventor: GETHINGS, Lee, Fulwood Preston PR2 9AT (GB); ISAAC, Giorgis, M., Marlborough, Massachusetts 01752 (US); MUNJOMA, Nyasha, Clarence, Cheshire Sale M33 3AB (GB); PLUMB, Robert, S., Milford, Massachusetts 01757 (US); RAINVILLE, Paul, Princeton, Massachusetts 01541 (US)
(74) Representative: Forresters IP LLP
(86) International application number: PCT/US2019/051907
(87) International publication number: WO 2020/061296

(56) References cited:
- WO-A1-2017/210097
- US-A1- 2017 160 264
- M. SCHERER ET AL: "A rapid and quantitative LC-MS/MS method to profile sphingolipids", THE JOURNAL OF LIPID RESEARCH, vol. 51, no. 7, 1 July 2010 (2010-07-01), pages 2001 - 2011, XP055071053, ISSN: 0022-2275, DOI: 10.1194/jlr.D005322

## Description

### CROSS-REFERENCE TO RELATED APPLICATIONS

This application claims the benefit of and priority to United States Provisional Patent Application No. 62/734,410 filed on September 21, 2018 titled "SYSTEM AND METHOD FOR LIPID QUANTIFICATION".

### FIELD OF THE TECHNOLOGY

The present disclosure generally relates to systems and methods for identifying and quantifying lipids. In particular, the present disclosure relates to systems and methods for identifying and quantifying lipids using a chromatography system coupled to a mass spectrometer.

### BACKGROUND

Chromatography involves the flowing of a mobile phase over a stationary phase to effect separation and is often combined with a detector, such as a mass spectrometer. Advances in MS have allowed for more in-depth analysis of lipids; however, unambiguous identification and quantification has proven difficult, as lipids exhibit a high number of isomeric and isobaric lipid species. Furthermore, MS spectra often contain peaks and fragments from multiple compounds, making confident identification and relative quantitation of specific molecular species difficult and time consuming. As a result, the transfer of lipidomic data between laboratories is severely hindered, making it problematic to draw biological interpretation with multiple-site studies.

WO2017/210097 discloses a mass spectrometry method for detection and quantitation of metabolites , US2017/160264 discloses the use of ceramides and lpls in diagnosing cvd. M. Scherer et al, "A rapid and quantitative LC-MS/MS method to profile sphingolipids", J. Lipid Res. 51(7), pages 2001-2011 (2010) discloses a method for simultaneous quantification of sphingolipid classes. Appropriate internal standards were added prior to lipid extraction.

### SUMMARY

Identification and quantification of lipids raises a number of challenges due to the high number of isomeric and isobaric lipid species. Technology for unambiguously and efficiently identifying and quantifying lipids would be beneficial and highly desirable.
The present invention provides a method for identifying and quantifying lipids in a biological sample comprising classes of lipids according to any one of claims 1 to 4, wherein any of these methods are used for identifying potential biomarkers according to claim 11, and for diagnostic screening for a medical condition associated with one or more lipids according to claim 12.

In one aspect, the present technology relates to a method for screening lipids. The method includes selecting a set of standards to identify at least one class of lipids; combining the standards with a biological sample to form a sample matrix; introducing the sample matrix into a chromatography system to separate the at least one class of lipids; directing the separated lipids to a detector; generating a calibration curve with known concentration of the set of standards; plotting a detector response of the separated lipids against the known concentrations of the set of standards; and quantifying the separated lipids based on a comparison between the detector response and the calibration curve. The detector is configured to perform targeted quantification of the separated lipids using multiple reaction monitoring (MRM) transitions. In another example embodiment, the MRM transitions are performed in both positive and negative ion mode. In another example embodiment, the MRM transitions can be fatty acyl chain fragments, head group fragments, or neutral loss fragments. In another example embodiment, the class of lipids includes monoradylglycerolipids (MG), diradylglycerolipids (DG), triradylglycerolipids (TG), ceramides, lysophosphatidylcholines (LPC), lysophosphatidylethanolamines (LPE), phosphatidylcholines (PC), sphingomyelins (SM), free fatty acids (FFA), lysophosphatidylinositols (LPI), phosphatidic acids (PA), lysophosphatidic acids (LPA), phosphatidylethanolamines (PE), phosphatidylglycerols (PG), phosphatidylinositols (PI), phosphatidylserines (PS), cholesterol, cholesterol ester, hexsocyl ceramides, dihexsocyl ceramides, lipoprotein(a) (LPA), lypopolysaccharides (LPS), or lysyl-phosphatidylglycerol (LPG). In another example embodiment, the chromatography system is a hydrophilic interaction chromatography (HILIC) system.

In another aspect, the present technology relates to a method for identifying potential biomarkers. The method includes selecting a set of standards to identify at least one class of lipids; combining the set of standards with a number of biological samples associated with a medical condition to form a sample matrix; introducing the sample matrix into a chromatography system to separate the at least one class of lipids; directing the separated lipids to a detector; generating a calibration curve with known concentrations of the set of standards; plotting a detector response of the separated lipids against the known concentrations of the set of standards; quantifying the separated lipids based on a comparison between the detector response and the calibration curve; and determining a relationship between the separated lipids and the medical condition.

The detector is configured to perform targeted quantification of the separated lipids using multiple reaction monitoring (MRM) transitions. In another example embodiment, the MRM transitions are performed in both positive and negative ion mode. In another example embodiment, the MRM transitions can be fatty acyl chain fragments, head group fragments, or neutral loss fragments. In another example embodiment, the class of lipids includes monoradylglycerolipids (MG), diradylglycerolipids (DG), triradylglycerolipids (TG), ceramides, lysophosphatidylcholines (LPC), lysophosphatidylethanolamines (LPE), phosphatidylcholines (PC), sphingomyelins (SM), free fatty acids (FFA), lysophosphatidylinositols (LPI), phosphatidic acids (PA), lysophosphatidic acids (LPA), phosphatidylethanolamines (PE), phosphatidylglycerols (PG), phosphatidylinositols (PI), phosphatidylserines (PS), cholesterol, cholesterol ester, hexsocyl ceramides, dihexsocyl ceramides, lipoprotein(a) (LPA), lypopolysaccharides (LPS), or lysyl-phosphatidylglycerol (LPG). In another example embodiment, the chromatography system is a hydrophilic interaction chromatography (HILIC) system.

Also disclosed, but not independently claimed, is a method for diagnostic screening. The method includes selecting a set of standards to identify at least one class of lipids, wherein an increased or decreased presence of the at least one class of lipids is indicative of a medical condition. The method also includes combining the set of standards with a biological sample to form a sample matrix; introducing the sample matrix into a chromatography system to separate the at least one class of lipids; and directing the separated lipids to a detector to determine an amount of the at least one class of lipids from the biological sample. The detector is configured to perform targeted quantification of the separated lipids using multiple reaction monitoring (MRM) transitions. In another example, the MRM transitions are performed in both positive and negative ion mode. In another example, the MRM transitions can be fatty acyl chain fragments, head group fragments, or neutral loss fragments. In another example, the class of lipids includes monoradylglycerolipids (MG), diradylglycerolipids (DG), triradylglycerolipids (TG), ceramides, lysophosphatidylcholines (LPC), lysophosphatidylethanolamines (LPE), phosphatidylcholines (PC), sphingomyelins (SM), free fatty acids (FFA), lysophosphatidylinositols (LPI), phosphatidic acids (PA), lysophosphatidic acids (LPA), phosphatidylethanolamines (PE), phosphatidylglycerols (PG), phosphatidylinositols (PI), phosphatidylserines (PS), cholesterol, cholesterol ester, hexsocyl ceramides, dihexsocyl ceramides, lipoprotein(a) (LPA), lypopolysaccharides (LPS), or lysyl-phosphatidylglycerol (LPG). In another example, the chromatography system is a hydrophilic interaction chromatography (HILIC) system.

The above aspects of the technology provide numerous advantages. In particular, examples of the techniques described herein provide rapid quantification of 106 choline containing phospholipids (61 PCs, 24 SMs and 21 LPCs), 24 ceramides, 23 hexosylceramides, 24 FFAs, and sphingosine in plasma and serum. Example embodiments disclosed herein also provide a high-throughput (as low as 8 minutes), quantitative and comprehensive LC/MS/MS method for the analysis of polar and non-polar lipid classes in plasma. Example embodiments can also provide methods for fast separation of 16 polar and non-polar lipid classes, and the quantitation of 504 lipid species from plasma samples (250 positive mode injection and 254 negative mode injection). Example embodiments disclosed herein can also improve identification and specificity of phosphatidylcholines (PC) using MRM transitions.

### BRIEF DESCRIPTION OF THE DRAWINGS

One of ordinary skill in the art will understand that the drawings primarily are for illustrative purposes and are not intended to limit the scope of the inventive subject matter described herein. The drawings are not necessarily to scale; in some instances, various aspects of the subject matter disclosed herein may be shown exaggerated or enlarged in the drawings to facilitate an understanding of different features. In the drawings, like reference characters generally refer to like features (e.g., functionally similar and/or structurally similar elements).
FIG. 1 shows an overview of an example HILIC-based comprehensive and high throughput non-polar and polar lipid class separation and quantification method compared to non-targeted approaches, according to an embodiment of the present disclosure.
FIG. 2A is a flowchart illustrating an exemplary method for screening lipids, according to an embodiment of the present disclosure.
FIG. 2B is a flowchart illustrating an exemplary method for identifying potential biomarkers, according to an embodiment of the present disclosure.
FIG. 2C is a flowchart illustrating an exemplary method for screening lipids, according to an embodiment of the present disclosure.
FIG. 3 is a graph showing the retention time reproducibility of 150 various injections of lipid standards using five columns from separate batches, according to an embodiment of the present disclosure.
FIG. 4 is a chromatogram of an example lipid standard mix, according to an embodiment of the present disclosure.
FIGS. 5A-5D are charts of exemplary lipid species and transitions for positive and negative plasma screens, according to an embodiment of the present disclosure.
FIG. 6 shows a chromatogram representing HILIC separation of PC, SM and LPC from the standard mixture in positive ion mode, according to an embodiment of the present disclosure.
FIG. 7 shows a structural representation of SM and PC.
FIG. 8 is a chromatogram showing the class separation of endogenous SMs and LPCs in plasma, thereby minimizing possible isobaric effects, according to an embodiment of the present disclosure.
FIG. 9A shows an example calibration curve for SIL standards LPC (18:1) (d7), according to an embodiment of the present disclosure.
FIG. 9B shows an example calibration curve for SM (18:1) (d9), according to an embodiment of the present disclosure.
FIGS. 9C-9D show example calibration curves representing PCs in positive and negative ion mode, respectively, according to an embodiment of the present disclosure.
FIG. 10 shows the structure of sphingolipids (SLs), ceramides (Cer), sphingosine 1-phosphate (S1P), sphingomyelin (SM) and hexosylceramide (HexCer), as well as the long-chain base (LCB) ion m/z 264, a characteristic product ion generated by SL.
FIG. 11A shows an overlaid chromatogram representing ceramides and sphingosine, according to an embodiment of the present disclosure.
FIG. 11B shows an overlaid positive ion mode chromatogram representing hexosylceramides species, according to an embodiment of the present disclosure.
FIG. 12 is a chromatogram showing TG and SM retention times compared to Cer, HexCer and SPH peaks, according to an embodiment of the present disclosure.
FIG. 13A shows a calibration curve for SM (18:1) (d9) (3-1500 ng/mL), according to an embodiment of the present disclosure.
FIG. 13B shows the calibration curve for TG (15:0/18:1/15:0) (d7) (5.5-2750 ng/mL, according to an embodiment of the present disclosure.
FIG. 14 is a positive ion mode chromatogram representing an example HILIC separation of a mixture of lipid standards, according to an embodiment of the present disclosure.
FIG. 15 shows representative chromatogram HILIC lipid class separations in positive ion mode from a plasma sample, according to an embodiment of the present disclosure.
FIGS. 16-19 show representative chromatogram HILIC lipid class separations in negative ion mode, according to an embodiment of the present disclosure.
FIG. 20 shows a representative calibration curve for 15:0/18:1(d7) PC in positive ion mode, according to an embodiment of the present disclosure.
FIG. 21 shows a representative calibration curve for 15:0/18:1(d7) PG in negative ion mode from a plasma sample, according to an embodiment of the present disclosure.
FIG. 22 shows the structure and nomenclature of different straight chain fatty acids.
FIG. 23 shows an overlaid chromatogram representing a HILIC separation of endogenous FFA lipids in plasma, according to an embodiment of the present disclosure.
FIG. 24 shows a representative calibration curve for PG (15:0/18:1) (d7) (16-8000 ng/mL), according to an embodiment of the present disclosure.
FIG. 25 shows a representative calibration curve for PC (15:0/18:1) (d7) (16-8000 ng/mL), according to an embodiment of the present disclosure.
FIGS. 26A-26D show chromatograms illustrating a choline head group, according to example embodiments of the present disclosure.
FIGS. 27A-27C show chromatograms illustrating a choline head group, according to example embodiments of the present disclosure.

The features and advantages of the present disclosure will become more apparent from the detailed description set forth below when taken in conjunction with the drawings.

### DETAILED DESCRIPTION

Following below are more detailed descriptions of various concepts related to, and embodiments of, methodologies, apparatus and systems for identifying and quantifying lipids. It should be appreciated that various concepts introduced above and discussed in greater detail below may be implemented in any of numerous ways, as the disclosed concepts are not limited to any particular manner of implementation. Examples of specific implementations and applications are provided primarily for illustrative purposes.

As used herein, the term "includes" means includes but is not limited to, the term "including" means including but not limited to. The term "based on" means based at least in part on.

The language "biological sample" refers to any solution or extract containing a molecule or mixture of molecules that comprises at least one biomolecule that is subjected to extraction or analysis that originated from a biological source (such as, humans and animals). Biological samples are intended to include crude or purified, e.g., isolated or commercially obtained, samples. Particular examples include, but are not limited to, inclusion bodies, biological fluids, biological tissues, biological matrices, embedded tissue samples, cells (e.g., one or more types of cells), and cell culture supernatants.

Unless specifically stated or obvious from context, the term "or," as used herein, is understood to be inclusive.

Lipids are essential in maintaining cell structures and play important roles in energy storage and cellular signaling. It is also known that lipids are important in the pathophysiology of diseases such as cancer, neurodegenerative diseases, infections, diabetes etc. Accordingly, the field of lipidomics has emerged when efforts have been devoted in identifying disease biomarkers and probing for disease mechanisms.

Lipids constitute a group of naturally occurring molecules that include fats, waxes, sterols, fat-soluble vitamins (such as vitamins A, D, E, and K), monoglycerides, diglycerides, triglycerides, phospholipids, and others. Major lipid categories include, such as, fatty acids, glycerolipids, glycerophospholipids, sphingolipids, saccharolipids, polyketides, sterol lipids, prenol lipids, fatty acids and their derivatives (including tri-, di-, monoglycerides, and phospholipids), as well as other sterol-containing metabolites such as cholesterol.

It is also appreciated that lipids can be classified in the following groups including, for example, monoradylglycerolipids (MG), diradylglycerolipids (DG), triradylglycerolipids (TG), ceramides, lysophosphatidylcholines (LPC), lysophosphatidylethanolamines (LPE), phosphatidylcholines (PC), sphingomyelins (SM), free fatty acids (FFA), lysophosphatidylinositols (LPI), phosphatidic acids (PA), phosphatidylethanolamines (PE), phosphatidylglycerols (PG), phosphatidylinositols (PI), phosphatidylserines (PS), cholesterol, Hexsocyl ceramides, lipoprotein(a) (LPA), lypopolysaccharides (LPS), and lysyl-phosphatidylglycerol (LPG).

Choline containing lipids such as phosphatidylcholine (PC) and sphingomyelin (SM) are a major component of cell membranes. PCs can account for more than 50% of the phospholipids in eukaryotic membranes. SMs are synthesized by the transfer of a phosphorylcholine group from PC to ceramide, forming the only nonglycerol phospholipid in cell membranes. Interest in measuring these lipid species has increased since they have been identified as being implicated in numerous diseases including multiple sclerosis and Niemann-Pick disease, for example.

Sphingolipids (SLs) are a class of lipid comprising of ceramides (Cer), sphingomyelins (SMs) and more complex glycosphingolipids. A common constituent of all SLs is a "long chain" or "sphingoid" base. These bases are organic aliphatic amino alcohols such as sphingosine (SPH) or a structurally similar compound. They are essential components of cellular membranes and are implicated in a variety of biological functions including cellular signal transduction and apoptosis. SLs can induce tumor cell proliferation and may be resistant to chemotherapy; Cer and hexosylceramide (HexCer) have been found to exhibit elevated levels in Parkinson's disease patients. The rapid metabolic interconversion of SLs as well as the high number of derivatives with similar functions, presents a challenge in terms of generating a profile of functions for individual sphingolipids.

Traditionally, lipids are only associated with cellular roles involving energy storage and used as structural building blocks. Recent developments in biomedical lipid research with the advance of mass spectrometric approach have identified the important role of lipids in different diseases or states including cancer, inflammation and cardiovascular diseases.

The extreme structural diversity of lipids in real biological samples is challenging for analytical techniques used in the lipidomics analysis due to the large difference in physicochemical properties (such as acidity, basicity, neutral, polar and non-polar) of individual lipid species. Currently the two main analytical strategies used for lipidomics are direct infusion MS and high performance reversed phase LC/MS. The direct infusion MS provides high-throughput analysis but can be less convenient for the separation and resolution of isobaric species and the identification of low abundant species because of possible ion suppression. Reversed-phase (RP)-LC is widely used for the separation of individual lipid molecular species based on their lipophilicity which is governed by the carbon chain length and number of double bonds. As a result, lipid species from different classes may co-elute. Normal-phase methods are also used for the separation of lipid classes using solvents such as hexane and chloroform, but their lengthy elution, limited analyte solubility and lack of solvent compatibility with mass spectrometer detection can make the technique less attractive.

Hydrophilic interaction chromatography (HILIC) is a type of normal-phase chromatography that can be used for the separation of lipid classes with a reversed phase solvent system to overcome these potential challenges. The lipid class separation using HILIC is convenient for LC/MS quantitation because lipid class stable isotope labelled (SIL) standards coelute with lipid species inside particular lipid classes under identical mobile phase and matrix composition. This provides identical ionization condition for the SIL standards and determined lipid class. An additional benefit of separating lipid species by class results in fewer SIL standards being required for quantification, thereby saving cost. This disclosure includes examples of HILIC-based comprehensive and high throughput non-polar and polar lipid class separation and quantitation.

A hydrophilic interaction chromatography (HILIC)-based method can offer a number of benefits. For example, a HILIC-based method can use solvents typically used for reversed-phase separations, which are compatible with electrospray ionization (ESI). That is, solvents, such as, acetonitrile, methanol, and water, can be used. Further, in a HILIC method, a highly organic mobile phase (e.g., >80% acetonitrile) can be used, which in turn results in an improved electrospray ionization through efficient mobile phase desolvation and compound ionization.

Fatty acids (FA) are hydrocarbon chains comprising of both carboxyl (-COOH) and methyl functional groups. Traditional nomenclature designates the carbon atom next to the carboxyl group as α and the subsequent one β carbon with the methyl group carbon designated ω. Fatty acid chains may contain one or more double bonds at specific positions (unsaturated and poly unsaturated with *cis* (Z) or *trans* (E) configuration) or they may be fully saturated. Branched chain fatty acids with one of more methyl branches on the carbon chain are more commonly found in prokaryotic organism but have been reported in cow's milk fat and the gastrointestinal tract of newborns.

Although free (non-esterified) fatty acids (FFA or NEFA) represent only a small fraction of total fatty acids in plasma, they represent a highly metabolically active lipid class. The most abundant FFAs in plasma consist of oleic acid (18:1), palmitic acid (16:0) and stearic acid (18:0) and together these make up 78% of all FFAs. Some FA species within the unsaturated family are long chain polyunsaturated fatty acids (LC-PUFAs) which are termed essential since they cannot be synthesized de novo. Examples include the main PUFAs, such as linoleic acid (18:2(n-6)), arachidonic acid (20:4(n-6)), alpha-linolenic acid (18:3(n-3)), gamma-linolenic acid (18:3(n-6)), eicosapentaenoic acid (20:5(n-6), EPA) and docosahexaenoic acid (22:6(n-3), DHA). Eicosanoids are locally acting bioactive signalling lipids derived from arachidonic acid and related polyunsaturated fatty acids (PUFAs) that regulate a diverse set of homeostatic and inflammatory processes linked to numerous diseases including metabolic syndrome disorders and cancers.

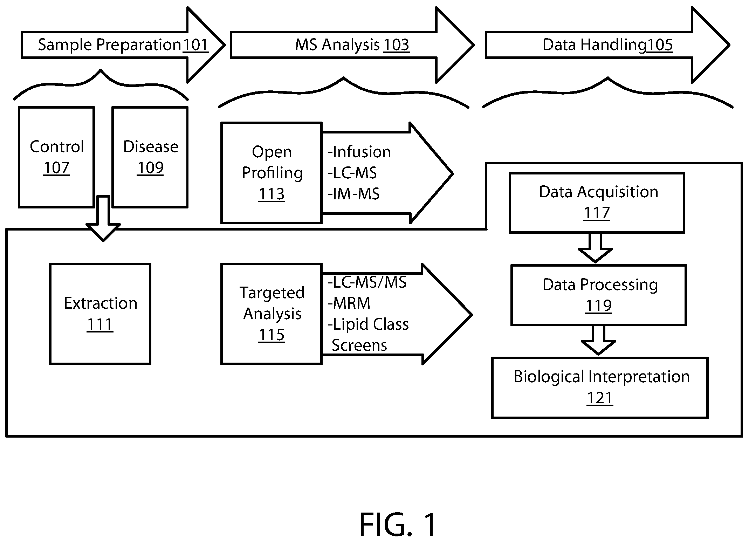

FIG. 1 shows an overview of an example HILIC-based comprehensive and high throughput non-polar and polar lipid class separation and quantification method, according to an embodiment of the present disclosure. In this non-limiting example, the method includes the higher level steps of sample preparation 101, MS analysis 103, and data handling 105. In this example, sample preparation 101 includes the preparation of a control sample 107, as well as a biological sample including a disease 109 or some medical condition of interest. An extraction method 111 is performed on both samples 107, 109. Once the sample preparation step 101 is completed, the MS analysis 103 is performed. Traditional open profiling 113 can include infusion, LC-MS analysis, and/or ion mobility (IM)-MS. The techniques described in this disclosure include a targeted analysis 115 that can include LC-MS/MS analysis, multiple reaction monitoring (MRM) transitions, and lipid class screens, in some embodiments. Once the MS analysis 103 is completed, data handling 105 can be performed. In a non-limiting example, data handling 105 includes the steps of data acquisition 117, data processing 119, and biological interpretation 121. In various embodiments, the techniques described herein can be applied to screen or identify lipids, identify biomarkers associated with a disease or medical condition, or as a method of diagnostic screening.

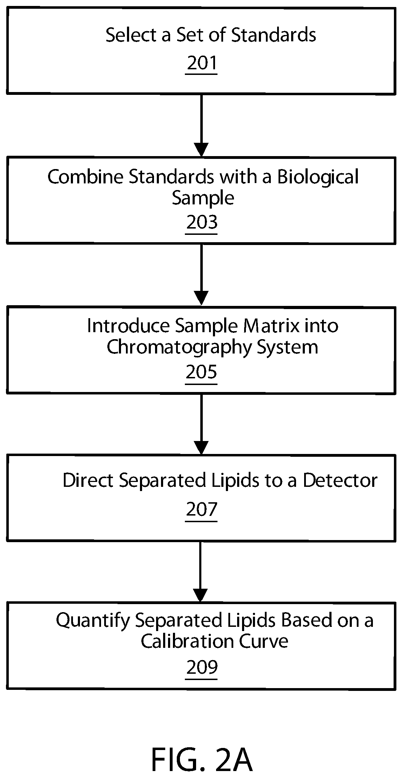

FIG. 2A is a flowchart illustrating an exemplary method for screening lipids, according to an embodiment of the present disclosure. In step 201, a set of standards is selected to identify at least one class of lipids. As discussed above, the class of lipids can include, for example, monoradylglycerolipids (MG), diradylglycerolipids (DG), triradylglycerolipids (TG), ceramides, lysophosphatidylcholines (LPC), lysophosphatidylethanolamines (LPE), phosphatidylcholines (PC), sphingomyelins (SM), free fatty acids (FFA), lysophosphatidylinositols (LPI), phosphatidic acids (PA), phosphatidylethanolamines (PE), phosphatidylglycerols (PG), phosphatidylinositols (PI), phosphatidylserines (PS), cholesterol, Hexsocyl ceramides, lipoprotein(a) (LPA), lypopolysaccharides (LPS), or lysyl-phosphatidylglycerol (LPG).

In step 203, the set of standards is combined with a biological sample to form a sample matrix. Once the sample matrix is formed, the sample matrix is introduced into a chromatography system in step 205 to separate the lipids. In a non-limiting example, the chromatography system can include a HILIC-based system. In some example embodiments, the lipids are extracted from the sample matrix before being introduced to the chromatography system. This step can be performed between steps 203 and 205, in some embodiments.

In step 207, the separated lipids are directed to a detector. In a non-limiting example, the detector is configured to perform a targeted quantification of the lipids using MRM transitions. In some embodiments, the MRM transitions can be performed in both positive and negative ion mode. In another non-limiting example, the MRM transitions can be fatty acyl chain fragments, head group fragments, or neutral loss fragments depending on the lipid class.

As used herein, an "acyl" group refers to a functional group derived by the removal of one or more hydroxyl groups from an oxoacid. The acyl group is usually derived from a carboxylic acid Therefore, it usually has the formula RCO-, where R represents an alkyl group that is attached to the CO group with a single bond.

In step 209, the separated lipids are quantified based on a comparison between detector data and a calibration curve generated with a known concentration of the set of standards. In some embodiments, the calibration curve can be generated prior, or calibration curve data can be retrieved from a database or library. A set of standards is prepared over the target concentration range and the detector response is plotted on the y-axis against the known concentrations of the lipid internal standards on the x-axis. Data is processed using TargetLynx or Skyline and least squares linear regression calculations used to determine the equation of the straight line with the best fit to the data. The equation is then used to convert a measured detector response for the biological sample to the predicted concentration for the biological sample.

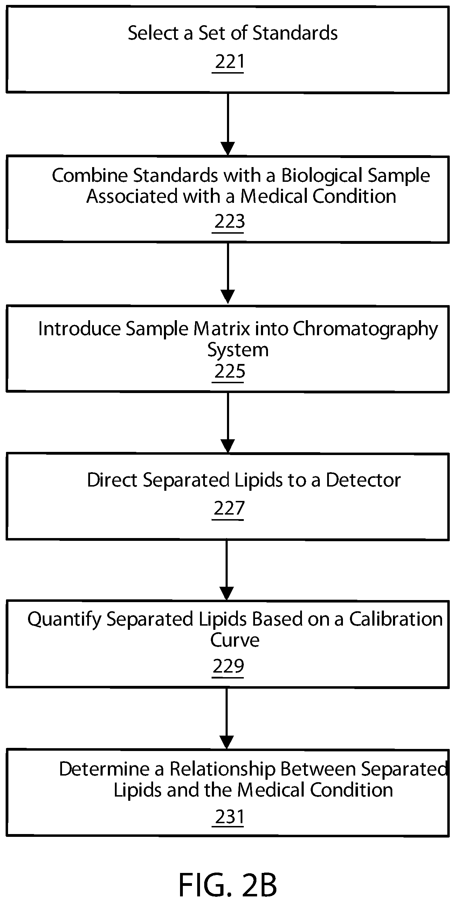

FIG. 2B is a flowchart illustrating an exemplary method for identifying potential biomarkers, according to an embodiment of the present disclosure. In step 221, a set of standards is selected to identify at least one class of lipids. As discussed above, the class of lipids can include, for example, monoradylglycerolipids (MG), diradylglycerolipids (DG), triradylglycerolipids (TG), ceramides, lysophosphatidylcholines (LPC), lysophosphatidylethanolamines (LPE), phosphatidylcholines (PC), sphingomyelins (SM), free fatty acids (FFA), lysophosphatidylinositols (LPI), phosphatidic acids (PA), phosphatidylethanolamines (PE), phosphatidylglycerols (PG), phosphatidylinositols (PI), phosphatidylserines (PS), cholesterol, Hexsocyl ceramides, lipoprotein(a) (LPA), lypopolysaccharides (LPS), or lysyl-phosphatidylglycerol (LPG).

In step 223, the set of standards is combined with a number of biological samples associated with a medical condition or a particular disease to form a sample matrix. Once the sample matrix is formed, the sample matrix is introduced into a chromatography system in step 225 to separate the lipids. In a non-limiting example, the chromatography system can include a HILIC-based system. In some example embodiments, the lipids are extracted from the sample matrix before being introduced to the chromatography system. This step can be performed between steps 223 and 225, in some embodiments.

In step 227, the separated lipids are directed to a detector. In a non-limiting example, the detector is configured to perform a targeted quantification of the lipids using MRM transitions. In some embodiments, the MRM transitions can be performed in both positive and negative ion mode. In another non-limiting example, the MRM transitions can be fatty acyl chain fragments, head group fragments, or neutral loss fragments depending on the lipid class.

In step 229, the separated lipids are quantified based on a comparison between detector data and a calibration curve generated with a known concentration of the set of standards. In some embodiments, the calibration curve can be generated prior, or calibration curve data can be retrieved from a database or library.

In step 231, a relationship between the separated lipids and the medical condition can be determined. For example, if the sample matrix formed in step 223 includes a biological sample associated with a particular disease or infection, and if the set of standards selected in step 221 are chosen to identify one particular class of lipids, if that particular class of lipids is detected and quantified at a significantly high rate, a determination could be made that there is a relationship between the detected class of lipids and the particular disease or infection in the biological sample. Such a technique can be used to identify various lipid biomarkers associated with a large number of medical conditions.

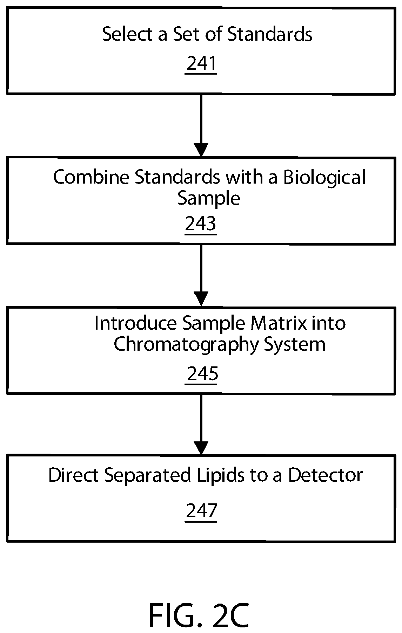

FIG. 2C is a flowchart illustrating an exemplary method for diagnostic screening, according to an embodiment of the present disclosure. In step 241, a set of standards is selected to identify at least one class of lipids, wherein an increased or a decreased presence of that class of lipids is indicative of a medical condition. As discussed above, the class of lipids can include, for example, monoradylglycerolipids (MG), diradylglycerolipids (DG), triradylglycerolipids (TG), ceramides, lysophosphatidylcholines (LPC), lysophosphatidylethanolamines (LPE), phosphatidylcholines (PC), sphingomyelins (SM), free fatty acids (FFA), lysophosphatidylinositols (LPI), phosphatidic acids (PA), phosphatidylethanolamines (PE), phosphatidylglycerols (PG), phosphatidylinositols (PI), phosphatidylserines (PS), cholesterol, Hexsocyl ceramides, lipoprotein(a) (LPA), lypopolysaccharides (LPS), or lysyl-phosphatidylglycerol (LPG).

In step 243, the set of standards is combined with a biological sample to form a sample matrix. In a non-limiting example, the biological sample can include a plasma sample from a patient, and the techniques described in FIG. 2C can be used as a method of screening the patient for a particular medical condition. Once the sample matrix is formed, the sample matrix is introduced into a chromatography system in step 245 to separate the lipids. In a non-limiting example, the chromatography system can include a HILIC-based system. In some example embodiments, the lipids are extracted from the sample matrix before being introduced to the chromatography system. This step can be performed between steps 243 and 245, in some embodiments.

In step 247, the separated lipids are directed to a detector to determine an amount of the class of lipids in the biological sample. In a non-limiting example, the biological sample can be a biological sample from a patient, and any increase or decrease of a particular class of lipids detected in step 247 can be indicative of a medical condition. As discussed above, the detector can be configured to perform a targeted quantification of the lipids using MRM transitions. In some embodiments, the MRM transitions can be performed in both positive and negative ion mode. In another non-limiting example, the MRM transitions can be fatty acyl chain fragments, head group fragments, or neutral loss fragments depending on the lipid class.

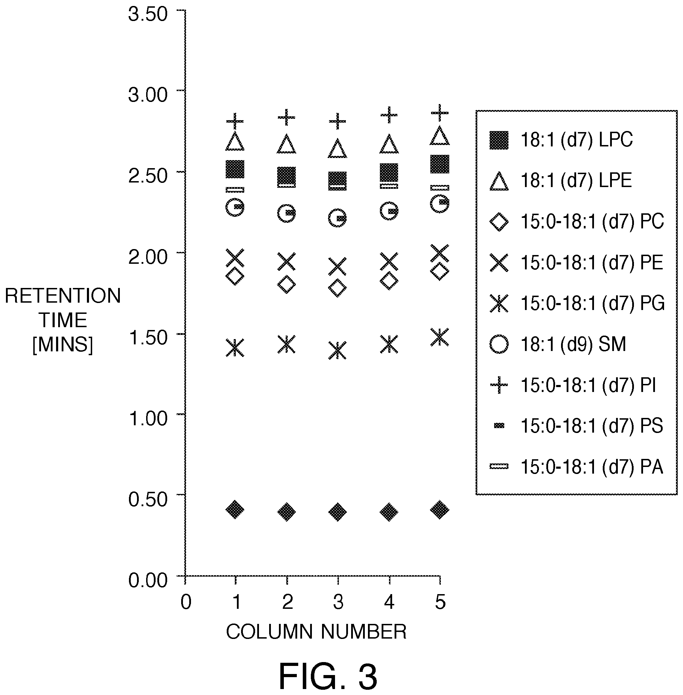

FIG. 3 is a graph showing the retention time reproducibility of 150 various injections of lipid standards using five columns from separate batches, according to an embodiment of the present disclosure. In this example experiment, the lipids tested included LPC, LPE, PC, PE, PG, SM, PI, PS, and PA and showed retention times RSD's of less than 1% (except for PG (d7) which was less than 2%).

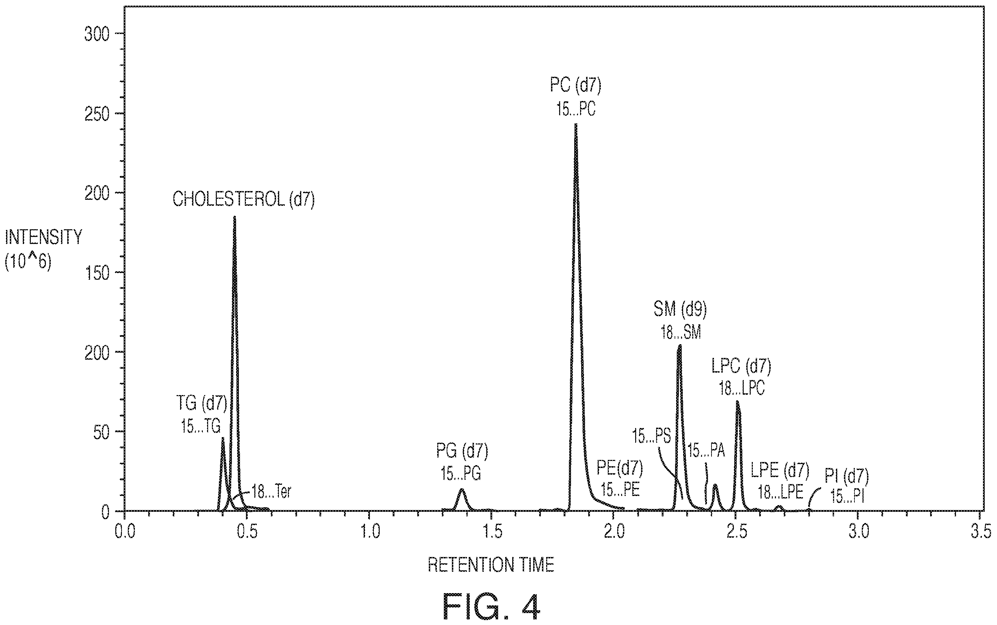

FIG. 4 is a chromatogram of an example lipid standard mix, according to an embodiment of the present disclosure. The LC conditions for this chromatogram include a mobile phase of 95:5 acetonitrile / water + 10 mM ammonium acetate and 50:50 acetonitrile / water + 10 mM ammonium acetate at 45 °C. Example MRM transitions used for the different lipid classes are shown below in Table 1.

**Table 1**

| **Lipid Class** | **Retention time (min)** | **Acquisition mode** | **Number of MRM transitions** |
|---|---|---|---|
| Cholesterol ester | 0.42 | +ve | 13 |
| MG | 0.50 | +ve | 25 |
| DG | 0.45 | +ve | 277 |
| TG | 0.41 | +ve | 21 |
| Ceramide | 0.90 | +ve | 47 |
| LPC | 2.08 | +ve | 22 |
| LPE | 2.25 | +ve | 11 |
| PC | 1.50 | +ve | 106 |
| SM | 1.85 | +ve | **25** |
| **Total** | | +ve | **547** |
| **Plasma Screen (+)** | | +ve | **250 (****FIG. 5A****)** |
| FFA | 0.51 | -ve | 24 |
| LPI | 2.40 | -ve | 24 |
| PA | 2.20 | -ve | 302 |
| PC | 1.50 | -ve | 278 |
| PE | 1.60 | -ve | 279 |
| PG | 1.20 | -ve | 219 |
| PI | 2.4 | -ve | 90 |
| PS | 2.15 | -ve | 278 |
| **Total** | | -ve | **1494** |
| **Plasma Screen (-)** | | -ve | **254 (****FIG. 5C****)** |
| **Mixture** | | +ve/-ve | **14** |

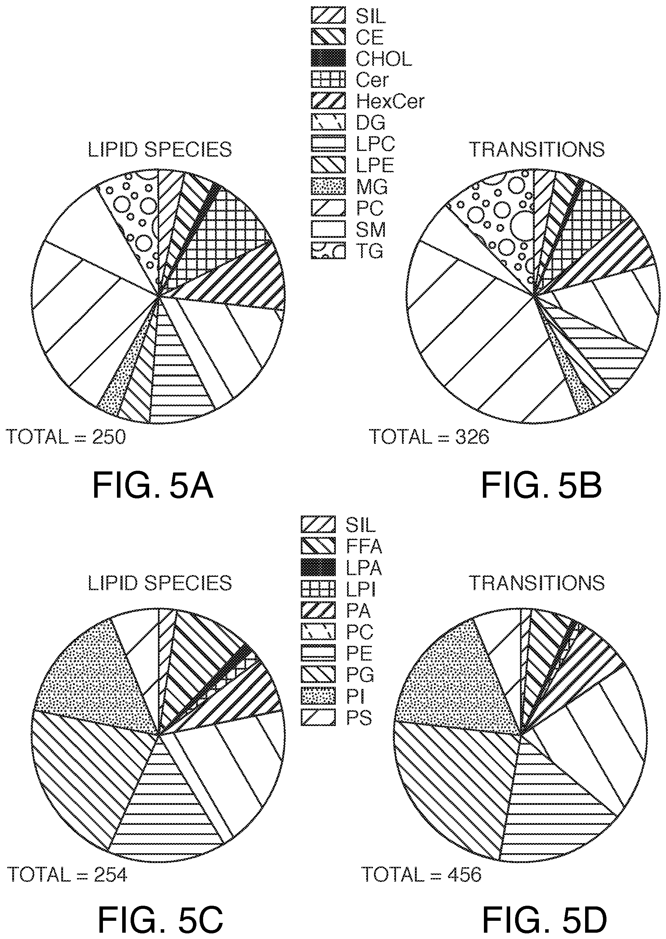

FIGS. 5A-5D are charts of exemplary lipid species and transitions for the positive and negative plasma screens described in Table 1, according to an embodiment of the present disclosure. FIG. 5A shows a pie chart of the lipid species identified (250) with the positive plasma screen, and FIG. 5B shows the total number of transitions (326) for the positive plasma screen. Similarly, FIG. 5C shows a pie chart of the lipid species identified (254) with the negative plasma screen, and FIG. 5D shows the total number of transitions 456) for the negative plasma screen.

### EXAMPLES

### Example 1

Example 1 includes an example HILIC-based LC-MS/MS high-throughput targeted phospholipids screen (PC, LPC, SM). This example illustrates the rapid quantification of 106 choline containing phospholipids (61 PCs, 24 SMs and 21 LPCs) in plasma and serum, as well as the improved identification and specificity of PC using MRM transitions from two fatty acyl chain fragments. A HILIC-based approach for the separation of lipids by class prior to MS analysis can reduce identification ambiguity. An additional benefit of separating lipid species by class results in fewer SIL standards being required for quantification, thereby saving cost.

In this example experiment, pooled healthy control plasma was spiked with SIL, (SPLASH LIPIDOMIX^{™}, Avanti Lipids, Alabaster, AL) at 9 concentration levels to generate calibration curves for quantification (PC (15:0-18:1) (d7) = 16-8000 ng/mL, SM (18:1) (d9) =3-1500 ng/mL and LPC (18:1) (d7) =2.5-1250 ng/mL). Six replicates of human plasma, which is commercially sold as NIST^{®} SRM^{®} 1950 (NIST U.S. Department of Commerce, Gaithersburg Maryland 20899), were also spiked with 5% SIL, prior to extraction.

A sample preparation procedure was adopted using protein precipitation with isopropanol (IPA) (1:5, plasma:IPA) and incubated for 2 hours. The extracted samples were centrifuged for 10 mins before transferring the supernatant to glass vials for LC/MS analysis. The LC conditions for the analysis are described below in Table 2, and the MS conditions for the analysis are described below in Table 3.

**Table 2**

| LC System | ACQUITY I-class UPLC system (Fixed Loop (FL) or Flow Through Needle (FTN)) |
|---|---|
| Column(s) | Acquity UPLC BEH Amide 2.1 x100 mm, 1.7 µm |
| Column Temp. | 45 °C |
| Flow Rate | 0.6 mL/minute |
| Mobile Phase | 95:5 Acetonitrile / Water + 10 mM Ammonium Acetate (A) and 50:50 Acetonitrile / Water + 10 mM Ammonium Acetate |
| Gradient | 0.1% to 20.0% B for 2 minutes, then 20% to 80% B for 3 minutes followed by 3 minutes re-equilibrium |
| Run Time | 8 Minutes |
| Injection Volume | 1 Microliter |

**Table 3**

| MS Systems | TQ-XS or TQ-S |
|---|---|
| Ionization Mode | ESI (+/-) |
| Capillary Voltage | 2.8 kV / 1.9 kV (-) |
| Acquisition Mode | MRM |
| Source Temperature | 120 °C |
| Desolvation Temperature | 500 °C |
| Cone Gas Flow | 150 L/hr |
| Desolvation Flow | 1000 L/hr |
| Nebuliser Gas | 7 bar |
| Ion Guide Offset 1 | 3 V |
| Ion Guide Offset 2 | 0.3 V |

In this example, a library was generated that contains the LC conditions, MS method, and associated processing method (including retention times). The resulting data can be processed using, for example, TargetLynx or Skyline (MacCoss Lab Software, University of Washington).

A rapid, specific LC-MS method was developed for the analysis of PC, LPC and SM lipids in human plasma employing a HILIC based chromatographic separation and MRM MS detection in both positive and negative ion mode. The HILIC methodology facilitated the elution of lipids in discrete classes. The resulting method was capable of measuring 61 PCs, 21 LPCs and 24 SMs in 8-minutes with a linear dynamic range over 4 orders of magnitude. The method sensitivity easily facilitated the detection of these lipids in human plasma at normal circulating levels from 50 µL of plasma.

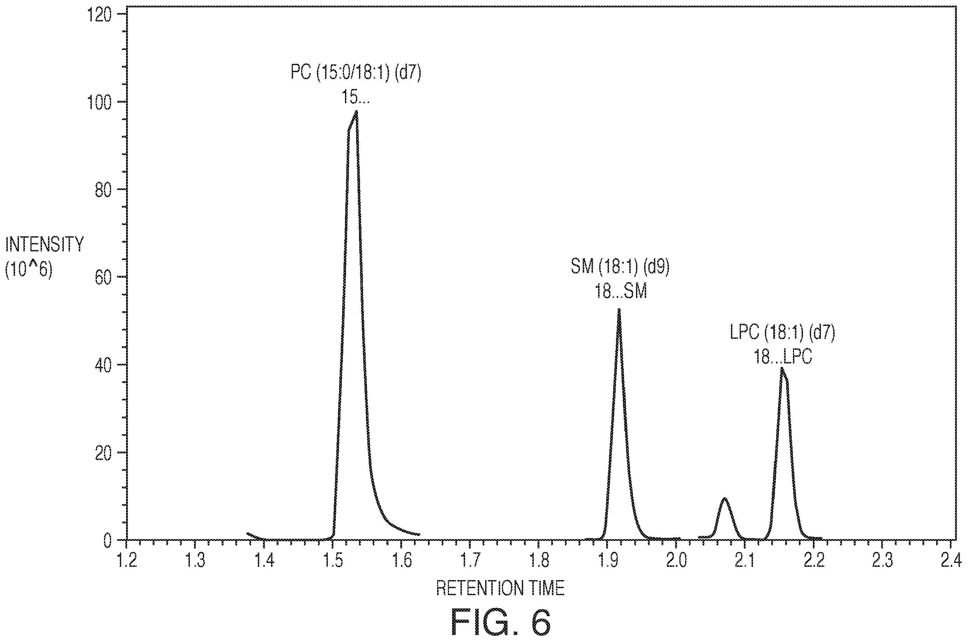

FIG. 6 shows a chromatogram representing HILIC separation of PC, SM and LPC from the standard mixture in positive ion mode, according to an embodiment of the present disclosure. As can be seen in this chromatogram, the HILIC methodology facilitated the elution of lipids in discrete classes, with the PCs eluting first (~1.52 mins) followed by SMs (~1.92 mins) and LPCs (~2.15 mins).

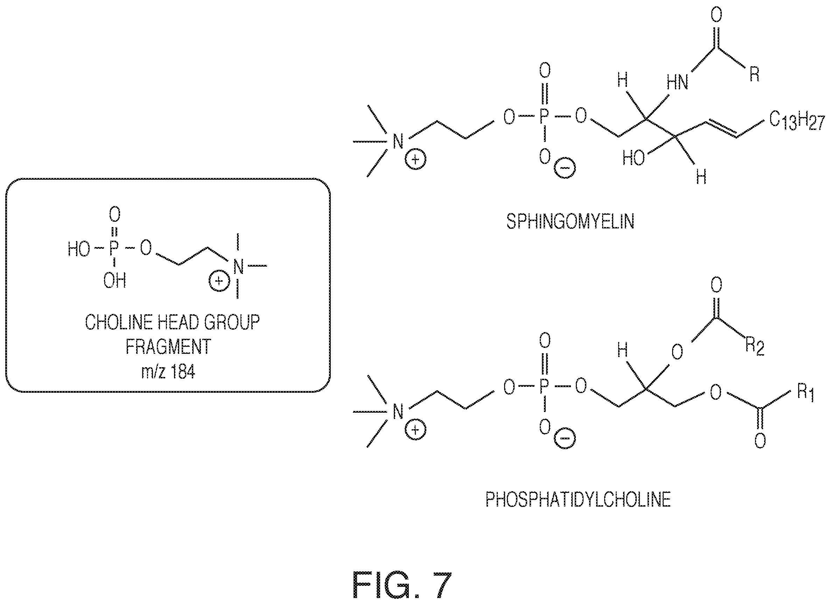

FIG. 7 shows a structural representation of SM and PC. Both species are choline-containing phospholipids that generate the characteristic phosphocholine head group fragment (m/z 184). Product ion scans of PC, SM, and LPC lipid classes showed the characteristic 184 m/z choline head group fragment in positive ion mode, which would result in unambiguous lipid assignment if employed in an MRM transition. To address this, highly specific MRM transitions were developed, containing fatty acyl chain fragments for 106 PCs (positive ion) and 278 PCs (negative ion) thereby improving identification and specificity.

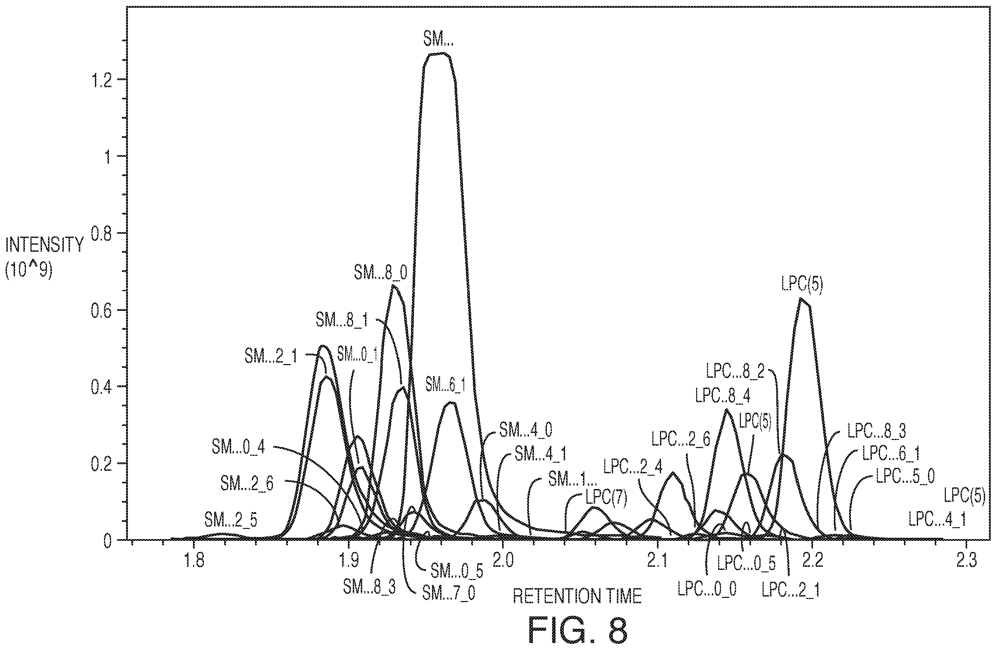

FIG. 8 is a chromatogram showing the class separation of endogenous SMs and LPCs in plasma, thereby minimizing possible isobaric effects, according to an embodiment of the present disclosure. Although the MRM transitions for LPC and SM species employ the choline head group fragment, chromatographic separation on the basis of class reduces isomeric and isobaric interferences. Additionally, isobaric effects are further minimized since LPC and SM precursors have mass ranges of 440-608 Da and 648-776 Da respectively, thus do not overlap.

The development of the library discussed above allows for the simple download of MRM transitions and chromatographic conditions representing PCs, LPCs and SMs, therefore eliminating manual input of LC-MS methods and reducing possible transcription errors.

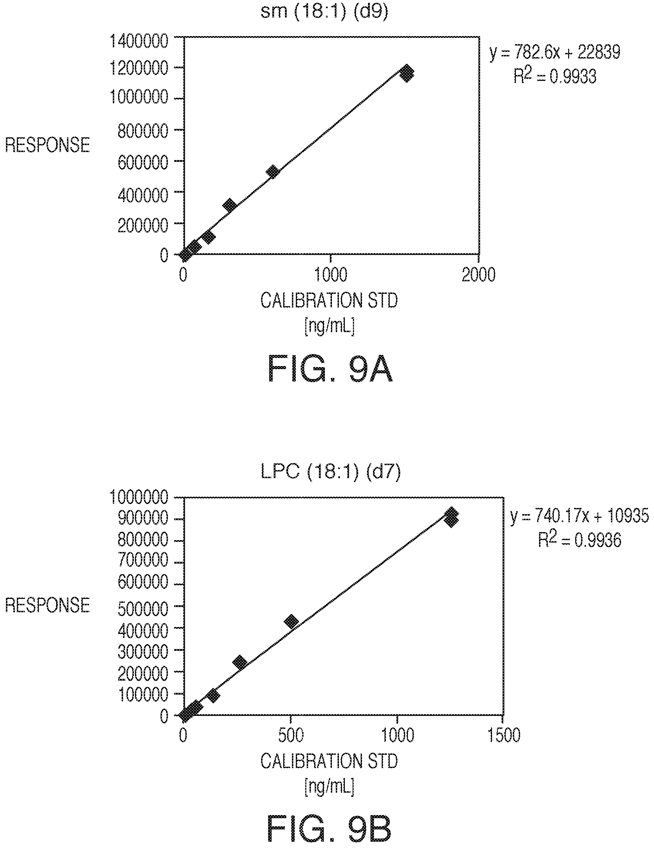

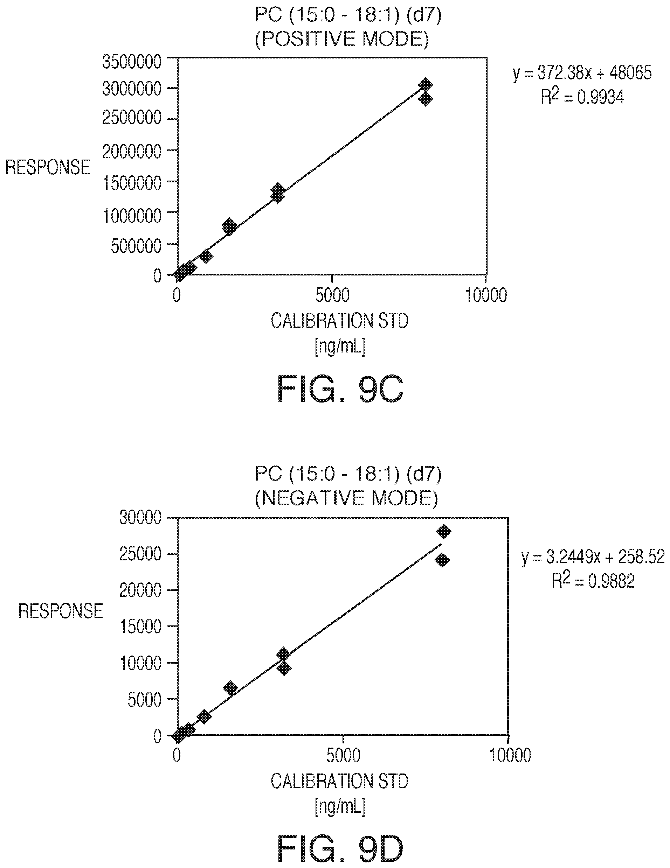

In this example embodiment, quantification was achieved using calibration curves of plasma spiked with known concentrations of SIL standards prior to extraction, providing surrogate standards for the quantification of endogenous lipids within the same class. By using surrogate standards prepared and analysed under identical conditions to those of endogenous lipids, the cost of large studies can be significantly reduced. Example calibration curves for SIL standards LPC (18: 1) (d7) and SM (18:1) (d9) are shown in FIGS. 9A-9B, which can be used for quantification of endogenous LPCs and SMs. Additional example curves representing PCs in positive and negative ion are also provided in FIGS. 9C-9D. Typical R² values of 0.95 or greater and deviations from the line of best fit (CVs <30%) are routinely achieved.

In this example, the method was shown to be linear over 4-orders of magnitude and had sufficient sensitivity to allow for the analysis of lipids at systemic levels in human plasma. Employing HILIC based chromatography allowed lipids to elute according to class, thereby reducing potential isomeric/isobaric interferences and the number of stable label isotopes required for quantification (i.e. cost reductions).

The concentrations of endogenous LPC lipids measured from this example are shown in Table 4. Readings with CVs <30% were considered acceptable. The back calculated spiked SIL results are included to show accuracy. The actual concentration of LPC (18: 1) (d7) spiked into the plasma was 1250 ng/mL.

**Table 4**

| **Calibration Range 2.5-1250 ng/mL** | RT [mins] | Average Conc [ng/mL] | Std Dev | CV [%] |
|---|---|---|---|---|
| LPC (18:1) (d7) | 2.15 | 1201.2 | 87.8 | 7.31 |
| LPC 16_0 | 2.20 | 32646.9 | 2471.4 | 7.57 |
| LPC 18_0 | 2.15 | 20200.4 | 1866.8 | 9.24 |
| LPC 18_1 | 2.16 | 13421.3 | 1278.3 | 9.52 |
| LPC 20_3 | 2.14 | 1213.1 | 118.6 | 9.77 |
| LPC 24_0 | 2.03 | 43.1 | 4.51 | 10.5 |
| LPC P-20_0 | 2.14 | 48.2 | 5.10 | 10.6 |
| LPC O- 18_0 | 2.17 | 965.4 | 102.8 | 10.6 |
| LPC 18_2 | 2.18 | 8881.5 | 1006.8 | 11.3 |
| LPC 18 3 | 2.20 | 194.3 | 23.6 | 12.1 |
| LPC O- 16_0 | 2.22 | 502.1 | 65.0 | 12.9 |
| LPC 22_1 | 2.18 | 128.5 | 17.9 | 14.0 |
| LPC 20 4 | 2.14 | 2740.8 | 384.7 | 14.0 |
| LPC 20_5 | 2.16 | 182.2 | 25.6 | 14.1 |
| LPC 20_0 | 2.10 | 77.4 | 11.1 | 14.3 |
| LPC 16_1 | 2.21 | 1508.9 | 223.6 | 14.8 |
| LPC 22_4 | 2.11 | 54.1 | 8.72 | 16.1 |
| LPC 22_6 | 2.13 | 399.7 | 70.9 | 17.7 |
| LPC P-18_1 | 2.04 | 15.9 | 2.96 | 18.5 |
| LPC 15_0 | 2.22 | 376.0 | 78.6 | 20.9 |

The concentrations of endogenous PC lipids measured from this example are shown in Table 5. Readings with CVs <30% were considered acceptable. The back calculated spiked SIL results are included to show accuracy. The actual concentration of PC (15:0-18:1) (d7) spiked into the plasma was 8000ng/mL

**Table 5**

| **Calibration Range 16-8000 ng/mL** | RT [mins] | Average Concentration [ng/mL] | Std Dev | CV [%] |
|---|---|---|---|---|
| PC (15:0-18:1) (d7) | 1.52 | 7918.8 | 782.5 | 9.88 |
| PC 18_1-18_2 | 1.49 | 49173.5 | 4484.2 | 9.12 |
| PC 18_1-18_1 | 1.48 | 25945.1 | 2382.1 | 9.18 |
| PC 16_0-20_4 | 1.47 | 74523.7 | 6874.4 | 9.22 |
| PC 18_0-18_2 | 1.49 | 150065.4 | 13950.1 | 9.30 |
| PC 16_0-18_1 | 1.50 | 47690.6 | 4492.5 | 9.42 |
| PC 16_0-22_4 | 1.47 | 3395.7 | 332.5 | 9.79 |
| PC 18_0-18_1 | 1.48 | 29513.9 | 2896.7 | 9.81 |
| PC 18_0-22_5 | 1.44 | 8000.4 | 793.8 | 9.92 |
| PC 16_0-20_3 | 1.48 | 22342.3 | 2291.8 | 10.3 |
| PC 16_0-22_6 | 1.46 | 18745.2 | 1947.4 | 10.4 |
| PC 16_0-22_5 | 1.46 | 9285.6 | 965.4 | 10.4 |
| PC 16_0-18_2 | 1.51 | 92445.9 | 9614.8 | 10.4 |
| PC 16_0-16_1 | 1.54 | 10368.2 | 1082.2 | 10.4 |
| PC 14_0-16_0 | 1.56 | 1682.8 | 175.7 | 10.4 |
| PC 18_2-18_2 | 1.49 | 13916.9 | 1469.9 | 10.6 |
| PC 16_1-18_1 | 1.50 | 1891.0 | 214.1 | 11.3 |
| PC 18_2-20_4 | 1.46 | 3986.7 | 456.6 | 11.5 |
| PC 18_0-20_3 | 1.46 | 15558.9 | 1788.3 | 11.5 |
| PC 16_0-16_0 | 1.54 | 15806.8 | 1847.1 | 11.7 |
| PC 16_0-18_0 | 1.50 | 4062.8 | 484.4 | 11.9 |
| PC 16_0-20_5 | 1.47 | 3139.8 | 383.9 | 12.2 |
| PC 18_0-20_5 | 1.45 | 3351.7 | 411.4 | 12.3 |
| PC 16_0-20_2 | 1.49 | 2547.6 | 324.8 | 12.8 |
| PC 18_1-20_2 | 1.47 | 778.3 | 99.4 | 12.8 |
| PC 18_0-20_2 | 1.47 | 1578.0 | 202.9 | 12.9 |
| PC 16_1-20_4 | 1.47 | 903.5 | 128.2 | 14.2 |
| PC 18_0-22_4 | 1.44 | 3088.5 | 471.0 | 15.2 |
| PC 16_0-18_3 | 1.51 | 1380.1 | 212.4 | 15.4 |
| PC 18_2-20_3 | 1.47 | 1330.6 | 206.7 | 15.5 |
| PC 16_1-18_2 | 1.51 | 1779.9 | 277.6 | 15.6 |
| PC 18_2-20_2 | 1.48 | 921.4 | 166.0 | 18.0 |
| PC 18_0-18_3 | 1.48 | 1520.2 | 302.7 | 19.9 |
| PC 18_1-20_1 | 1.47 | 469.2 | 94.9 | 20.2 |
| PC 16_1-18_0 | 1.50 | 933.2 | 193.2 | 20.7 |
| PC 18_1-18_3 | 1.49 | 459.9 | 96.7 | 21.0 |
| PC 16_0-20_1 | 1.49 | 370.6 | 85.1 | 23.0 |
| PC 16_1-20_3 | 1.48 | 357.2 | 82.9 | 23.2 |
| PC 18_2-20_0 | 1.46 | 643.7 | 169.0 | 26.2 |
| PC 16_0-22_3 | 1.46 | 280.6 | 78.2 | 27.9 |
| PC 18_1-20_0 | 1.45 | 289.5 | 80.7 | 27.9 |

The concentrations of endogenous SM lipids measured from this example are shown in Table 6. Readings with CVs <30% were considered acceptable. The back calculated spiked SIL results are included to show accuracy. The actual concentration of SM (18:1) (d9) spiked into the plasma was 1500ng/mL.

**Table 6**

| **Calibration Range 3-1500 ng/mL** | RT [mins] | Average Conc [ng/mL] | Std Dev | CV [%] |
|---|---|---|---|---|
| SM (18:1) (d9) | 1.92 | 1629.2 | 138.4 | 8.50 |
| SM d18_1-16_0 | 1.96 | 55832.2 | 2131.3 | 3.82 |
| SM d18_1-22_0 | 1.89 | 19988.1 | 845.1 | 4.23 |
| SM d18_1-22_1 | 1.89 | 14574.3 | 644.7 | 4.42 |
| SM d18_1-22_4 | 1.89 | 25.3 | 1.53 | 6.06 |
| SM d18_1-22_3 | 1.90 | 183.0 | 11.8 | 6.46 |
| SM d18_1-22_2 | 1.90 | 1255.1 | 82.2 | 6.55 |
| SM d18_1-20_0 | 1.91 | 8023.8 | 550.5 | 6.86 |
| SMd18_1-20_1 | 1.91 | 4043.3 | 300.7 | 7.44 |
| SM d18_1-16_1 | 1.96 | 11173.3 | 841.2 | 7.53 |
| SMd18_1-18_1 | 1.93 | 9128.8 | 690.8 | 7.57 |
| SM d18_1-17_0 | 1.94 | 1959.3 | 148.5 | 7.58 |
| SMd18_1-18_0 | 1.93 | 15673.5 | 1198.5 | 7.65 |
| SM d18_1-18_3 | 1.93 | 81.8 | 6.36 | 7.78 |
| SM d18_1-20_3 | 1.91 | 72.4 | 5.65 | 7.80 |
| SM d18_1-20_2 | 1.90 | 431.8 | 34.3 | 7.95 |
| SMd18_1-18_2 | 1.93 | 1141.6 | 91.7 | 8.03 |
| SM d18_1-14_0 | 1.99 | 4011.3 | 349.4 | 8.71 |
| SMd18_1-22_6 | 1.89 | 188.2 | 17.2 | 9.12 |
| SM d18_1-14_1 | 2.00 | 399.4 | 43.3 | 10.8 |
| SMd18_1-12_0 | 2.02 | 196.6 | 22.1 | 11.2 |
| SMd18_1-20_4 | 1.90 | 8.46 | 1.10 | 13.0 |
| SM d18_1-22_5 | 1.89 | 8.16 | 1.11 | 13.6 |
| SM d18_1-18_4 | 1.96 | 4.07 | 0.67 | 16.5 |

### Example 2

Example 2 includes an example HILIC-based LC-MS/MS high-throughput targeted ceramide and hexosylceramide screen. This example illustrates the rapid quantification of 24 ceramides, 23 hexosylceramide and sphingosine in plasma and serum. As discussed above HILIC-based approach for the separation of lipids by class prior to MS analysis can reduce identification ambiguity. An additional benefit of separating lipid species by class results in fewer SIL standards being required for quantification, thereby saving cost. This example describes the use of a HILIC based approach to perform a targeted screen for Cer, HexCer and SPH.

Pooled healthy control plasma was spiked with SIL, (SPLASH LIPIDOMIX^{™}, Avanti Lipids, Alabaster, AL) at 9 concentration levels to generate calibration curves for quantification. SPLASH LIPIDOMIX^{™} does not contain a suitable surrogate standard for the quantification of Cer or HexCer, but the linearity data can be used to assess the quality of the data generated. Typical R² values of 0.95 and deviations from the line of best fit (CVs <30%) are routinely achieved. SM (18:1) (d9) = 3-1500 ng/mL and TG (15:0/18:1/15:0) (d7) = 5.5-2750 ng/mL are used to illustrate this. Six replicates of the NIST^{®} SRM^{®} 1950 were also spiked with 5% SIL, prior to extraction.

A simple sample preparation procedure was adopted using protein precipitation with isopropanol (IPA) (1:5, plasma:IPA) and incubated for 2 hours. The extracted samples were centrifuged for 10 mins before transferring the supernatant to glass vials for LC-MS analysis. The LC conditions for the analysis are described below in Table 7, and the MS conditions for the analysis are described below in Table 8.

**Table 7**

| | |
|---|---|
| LC System | ACQUITY I-class UPLC system (Fixed Loop (FL) or Flow Through Needle (FTN)) |
| Column(s) | Acquity UPLC BEH Amide 2.1 x100 mm, 1.7 µm |
| Column Temp. | 45 °C |
| Flow Rate | 0.6 mL/minute |
| Mobile Phase | 95:5 Acetonitrile / Water + 10 mM Ammonium Acetate (A) and 50:50 Acetonitrile / Water + 10 mM Ammonium Acetate (B) |
| Gradient | 0.1% to 20.0% B for 2 minutes, then 20% to 80% B for 3 minutes followed by 3 minutes re-equilibrium |
| Run Time | 8 Minutes |
| Injection Volume | 1 Microliter |

**Table 8**

| | |
|---|---|
| MS Systems | TQ-XS or TQ-S |
| Ionization Mode | ESI (+) |
| Capillary Voltage | 2.8 kV (+) |
| Acquisition Mode | MRM |
| Source Temperature | 120 °C |
| Desolvation Temperature | 500 °C |
| Cone Gas Flow | 150 L/hr |
| Desolvation Flow | 1000 L/hr |
| Nebuliser Gas | 7 bar |
| Ion Guide Offset 1 | 3 V |
| Ion Guide Offset 2 | 0.3 V |

In this example, a library was generated containing the LC conditions, MS method, and associated processing method (including retention times). The resulting data were with either TargetLynx or Skyline (MacCoss Lab Software, University of Washington).

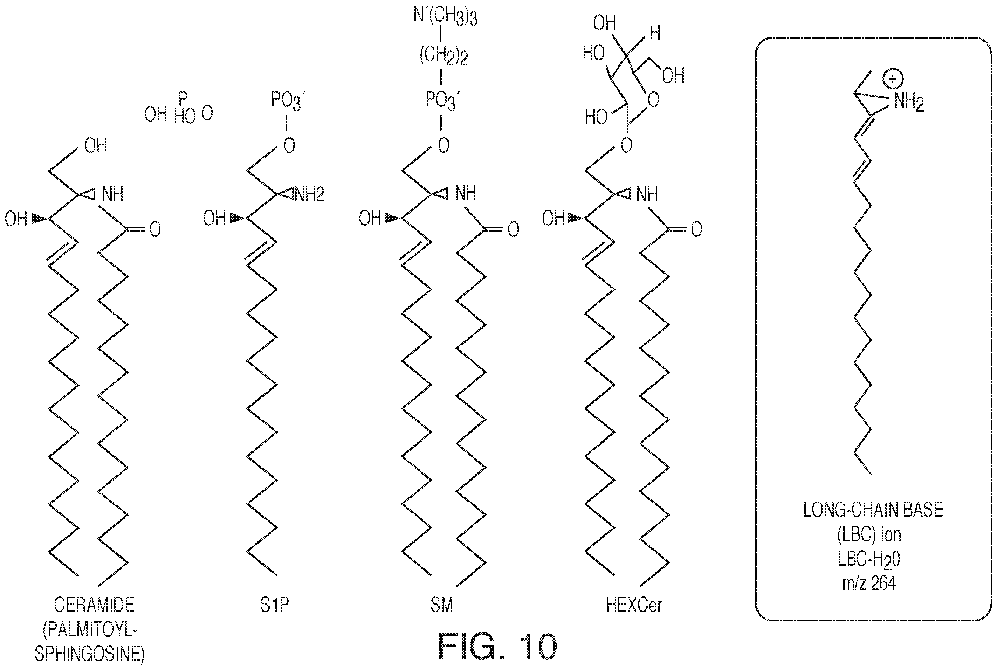

FIG. 10 shows the structure of sphingolipids (SLs), ceramides (Cer), sphingosine 1-phosphate (S1P), sphingomyelin (SM) and hexosylceramide (HexCer). The insert shows the long-chain base (LCB) ion m/z 264, a characteristic product ion generated by SL.

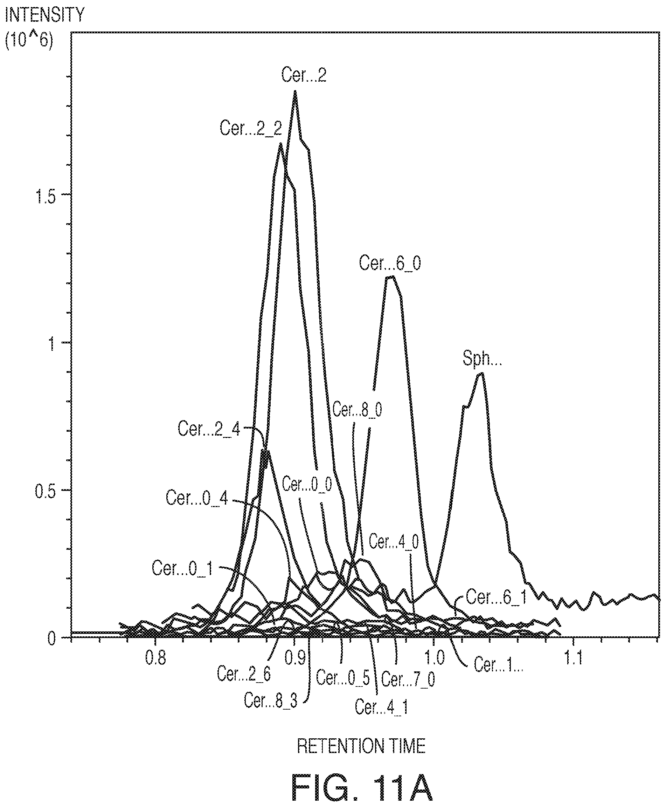

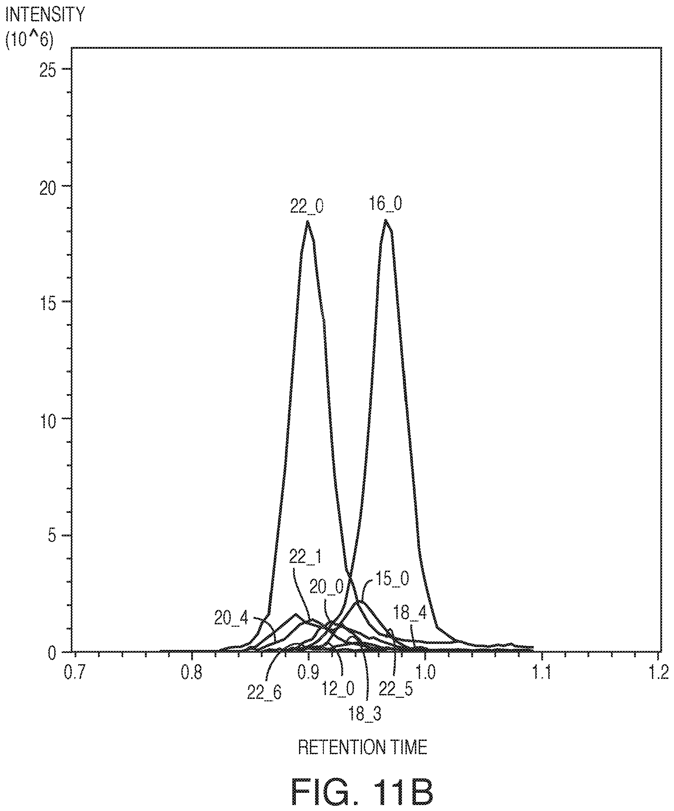

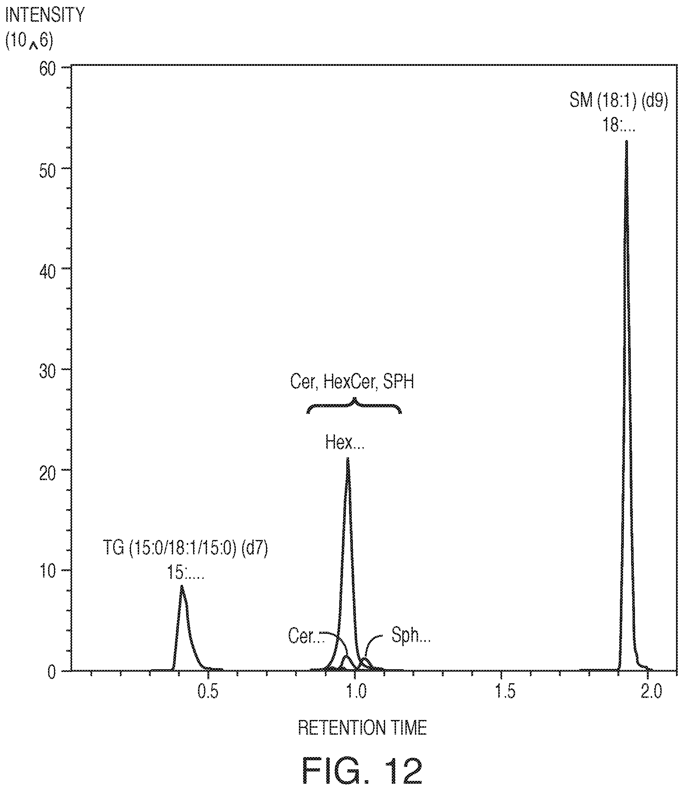

FIG. 11A shows an overlaid chromatogram representing ceramides and sphingosine. FIG. 11B shows an overlaid chromatogram representing hexosylceramides species (positive ion mode). FIG. 12 is a chromatogram showing TG and SM retention times compared to Cer, HexCer and SPH peaks, according to an embodiment of the present disclosure.

This example experiment employs MRM in positive ion mode to identify and quantify Cer and HexCer species contained in human plasma while simultaneously analysing several other lipid classes such as TG, PC and SM. Cer, HexCer and SPH lipids elute as a discrete band (~0.9-1.0 mins) under HILIC based conditions. A total of 24 Cer, 23 HexCer and SPH were analysed. The method sensitivity easily facilitated the detection of these lipids in human plasma at normal circulating levels from 50 µL of plasma, while demonstrating a linear dynamic range over 4 orders of magnitude.

The Cer and HexCer lipid transitions discussed here use the lipid precursor ion and characteristic long chain base (LCB) product ion (m/z 264). Although the peaks of Cer and HexCer species co-elute under these chromatographic conditions the risk of isobaric effects are minimal since Cer and HexCer precursors have mass ranges of 482.5-610.5 Da and 644.5-772.6 Da respectively. The choline containing SMs produce a characteristic 184 m/z head group fragment and elute at ~1.92 mins, enabling their analysis alongside other SLs with minimal risk of isomeric/isobaric interferences. Quantification of endogenous SMs is discussed in a separate application note [8] that includes other choline containing lipids (LPC and PC).

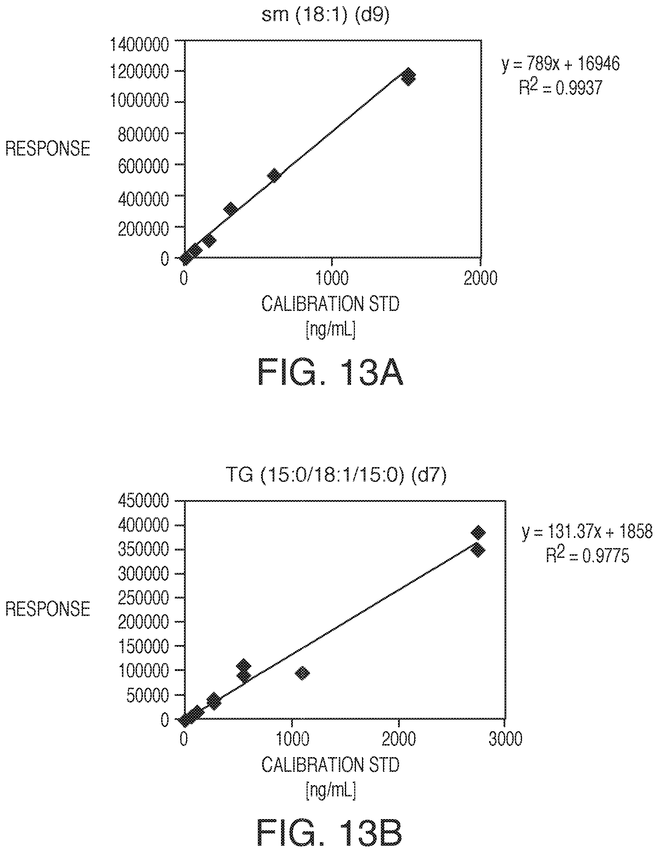

FIG. 13A shows a calibration curve for SM (18:1) (d9) (3-1500 ng/mL). FIG. 13B shows the calibration curve for TG (15:0/18:1/15:0) (d7) (5.5-2750 ng/mL). Typical R² values of >0.95 and deviations from the line of best fit (CV <30%) are specified as acceptable criteria. The data generated using the curated HILIC-based quantification method plasma screens are shown below in Tables 9 and 10. Average peak area responses for Cer and HexCer species with CVs <30% are shown. Typical R² values of 0.95 and deviations from the line of best fit (CVs <30%) are routinely achieved using the available SIL SPLASH^{®} LIPIDOMIX^{®} standard (Avanti Lipids, Inc. 700 Industrial Drive, Alabaster AL, 35007). The calibration curve for SM (18:1) (d9) = 0.5-1500 ng/mL and TG (15:0/18:1/15:0) (d7) = 5.5-2750 ng/mL illustrate this. TG (15:0/18:1/15:0) (d7) is the closest eluting SIL peak to the Cer, HexCer and SPH peaks. SM (18:1) (d9) is considered a SL but elutes ~ 1 min away from Cer and HexCer.

The development of the library discussed above allows for the simple download of MRM transitions and chromatographic conditions representing Cer and HexCer lipid species, therefore eliminating manual input of LC-MS methods and reducing possible transcription errors

Table 9 shows the area response of endogenous ceramides lipids in NIST^{®} SRM^{®} 1950 plasma. Readings with CVs <30% were considered acceptable.

**Table 9**

| **Ceramides** | RT [mins] | MRM Transition | Average Responses | Std Dev | CV [%] |
|---|---|---|---|---|---|
| Cer d18_1-22_0 | 0.90 | 622.6>264.3 | 67079.2 | 8355.0 | 12.5 |
| Cer d18_1-16_0 | 0.97 | 538.5>264.3 | 42746.7 | 5415.2 | 12.7 |
| Cer d18_1-18_0 | 0.95 | 566.6>264.3 | 6405.5 | 856.7 | 13.4 |
| Cer d18_1-22_5 | 0.89 | 612.5>264.3 | 4993.0 | 680.4 | 13.6 |
| Cer d18_1-22_2 | 0.89 | 618.6>264.3 | 59524.4 | 8151.4 | 13.7 |
| Cer d18_1-22_4 | 0.88 | 614.6>264.3 | 22168.2 | 3132.0 | 14.1 |
| Cer d18_1-22_1 | 0.90 | 620.6>264.3 | 5756.7 | 889.7 | 15.5 |
| Cer d18_1-20_4 | 0.90 | 586.5>264.3 | 6237.9 | 1049.5 | 16.8 |
| Cer d18_1-22_3 | 0.89 | 616.6>264.3 | 2603.0 | 452.4 | 17.4 |
| Cer d18_1-20_0 | 0.93 | 594.6>264.3 | 7125.1 | 1371.9 | 19.3 |
| Cer d18_1-20_2 | 0.91 | 590.6>264.3 | 2067.0 | 434.9 | 21.0 |
| Cer d18_1-18_4 | 0.92 | 558.5>264.3 | 1321.0 | 287.5 | 21.8 |
| Cer d18_1-22_6 | 0.90 | 610.5>264.3 | 711.2 | 156.8 | 22.0 |
| Cer d18_1-18_1 | 0.98 | 564.5>264.3 | 1339.6 | 362.2 | 27.0 |
| Cer d18_1-18_2 | 0.94 | 562.5>264.3 | 360.7 | 103.2 | 28.6 |
| | | | | | |
| Sphingosine d18_1 | 1.03 | 300.3>282.3 | 19605.2 | 2973.7 | 15.2 |

Table 10 shows the area response of endogenous hexosylceramides lipids in NIST^{®} SRM^{®} 1950 plasma. Readings with CVs <30% were considered acceptable.

**Table 10**

| **Hexosylceramides** | RT [mins] | MRM Transition | Average Responses | Std Dev | CV [%] |
|---|---|---|---|---|---|
| HexCer d18_1-22_0 | 0.91 | 784.7>264.3 | 722004.1 | 83039.4 | 11.5 |
| HexCer d18_1-22_2 | 0.90 | 780.6>264.3 | 98634.6 | 12262.6 | 12.4 |
| HexCer d18_1-22_1 | 0.91 | 782.7>264.3 | 56035.7 | 6989.6 | 12.5 |
| HexCer d18_1-18_0 | 0.95 | 728.6>264.3 | 73062.2 | 9183.7 | 12.6 |
| HexCer d18_1-14_0 | 1.00 | 672.5>264.3 | 4015.0 | 506.6 | 12.6 |
| HexCer d18_1-20_2 | 0.91 | 752.6>264.3 | 5798.6 | 762.4 | 13.1 |
| HexCer d18_1-20_0 | 0.93 | 756.6>264.3 | 48822.4 | 6504.5 | 13.3 |
| HexCer d18_1-18_1 | 0.95 | 726.6>264.3 | 12574.3 | 1678.7 | 13.4 |
| HexCer d18_1-16_1 | 0.97 | 698.6>264.3 | 3075.2 | 422.7 | 13.7 |
| HexCer d18_1-16_0 | 0.97 | 700.6>264.3 | 637913.8 | 88733.6 | 13.9 |
| HexCer d18_1-20_1 | 0.92 | 754.6>264.3 | 4378.4 | 618.0 | 14.1 |
| HexCer d18_1-18_2 | 0.94 | 724.6>264.3 | 2226.4 | 335.3 | 15.1 |
| HexCer d18_1-22_3 | 0.90 | 778.6>264.3 | 3127.4 | 483.7 | 15.5 |
| HexCer d18_1-22_6 | 0.91 | 772.6>264.3 | 630.7 | 99.7 | 15.8 |

In this example experiment, a rapid quantitative method was demonstrated for the analysis of SLs in conjunction with other lipid classes for plasma and serum. The methodology allowed for the analysis of 24 ceramides, 23 hexosylceramides, and SPH within 8-minutes. The method was shown to be linear over 4-orders of magnitude and had sufficient sensitivity to allow for the analysis of lipids at systemic levels in human plasma.

### Example 3

Example 3 includes an example HILIC-based LC-MS/MS high-throughput (as low as 8 minutes or lower) targeted lipid quantitation platform for the analysis of polar and non-polar lipid classes in plasma. This example illustrates the rapid quantitation of 504 lipid species from plasma sample (250 positive mode injection and 254 negative mode injection).

In this example, pooled healthy control plasma was spiked with SIL standards, (SPLASH^{®} LIPIDOMIX^{®}, Avanti Lipids, Alabaster, AL) at 9 concentration levels to generate calibration curves for quantification. Table 13 below shows the list of SIL standards spiked into the NIST^{®} SRM^{®} 1950 plasma extract and other calibration parameters in positive and negative ion mode. Six replicates of the NIST^{®} SRM^{®} 1950 were also spiked with 5% SIL, prior to extraction.

A simple sample preparation procedure was adopted using protein precipitation with isopropanol (IPA) (1:5, plasma:IPA) and incubated for 2 hours. The extracted samples were centrifuged for 10 mins before transferring the supernatant to glass vials for LC/MS analysis. The LC conditions for the analysis are described below in Table 11, and the MS conditions for the analysis are described below in Table 12.

**Table 11**

| | |
|---|---|
| LC System | ACQUITY I-class UPLC system (Fixed Loop (FL) or Flow Through Needle (FTN)) |
| Column(s) | Acquity UPLC BEH Amide 2.1 x100 mm, 1.7 µm |
| Column Temp. | 45 °C |
| Flow Rate | 0.6 mL/minute |
| Mobile Phase | 95:5 Acetonitrile / Water + 10 mM Ammonium Acetate (A) and 50:50 Acetonitrile / Water + 10 mM Ammonium |
| | Acetate (B) |
| Gradient | 0.1% to 20.0% B for 2 minutes, then 20% to 80% B for 3 minutes followed by 3 minutes re-equilibrium |
| Run Time | 8 Minutes |
| Injection Volume | 1 Microliter |

**Table 12**

| | |
|---|---|
| MS Systems | TQ-XS or TQ-S |
| Ionization Mode | ESI (+/-) |
| Capillary Voltage | 2.8 kV / 1.9 kV (-) |
| Acquisition Mode | MRM |
| Source Temperature | 120 °C |
| Desolvation Temperature | 500 °C |
| Cone Gas Flow | 150 L/hr |
| Desolvation Flow | 1000 L/hr |
| Nebuliser Gas | 7 bar |
| Ion Guide Offset 1 | 3 V |
| Ion Guide Offset 2 | 0.3 V |

In this example, a library was generated containing the LC conditions, MS method, and associated processing method (including retention times). The resulting data were processed with either TargetLynx or Skyline (MacCoss Lab Software, University of Washington).

Lipidomics involves the analysis of large scale sample sets, and therefore a high throughput method is desirable. A rapid and comprehensive LC-MS method was developed for the analysis of polar and non-polar lipid classes in human plasma employing a HILIC based lipid class separation and MRM MS quantitation in both positive and negative ion mode. Table 13 shows the list of SIL standards spiked into the NIST 1950 plasma extract and calibration parameters in positive and negative ion mode. The different lipid classes measured with their corresponding MRM retention time window, acquisition mode used, and the total number of MRM transitions is shown above in Table 1. A total of 2041 lipid species MRM transition can be measured from 16 polar and non-polar lipid classes with 547 in positive mode and 1494 in negative mode. A mixture of SIL lipid standards representing different lipid classes were used to demonstrate the separation of the lipid classes.

**Table 13**

| **SIL standard** | **Retention time (min)** | **Acquisition mode** | **Calibration Range (ng/mL)** | **Correlation Coefficient** |
|---|---|---|---|---|
| 18:1(d7) Cholesterol ester | 0.43 | +ve | 35.00-17500 | - |
| Cholesterol (d7) | 0.46 | +ve | 10.00-5000 | - |
| 18:1(d7) MG | 0.50 | +ve | 0.20-100 | - |
| 15:0/18:1(d7) DG | 0.45 | +ve | 1.00-500 | 0.972 |
| 15:0/18:1(d7)/15:0 TG | 0.40 | +ve | 5.50-2750 | 0.989 |
| 18:1(d7) LPC | 2.08 | +ve | 2.50-1250 | 0.994 |
| 18:1(d7) LPE | 2.27 | +ve | 0.50-250 | 0.988 |
| 15:0/18:1(d7) PC | 1.50 | +ve | 16.00-8000 | 0.993 |
| 18:1/18:1(d9) SM | 1.88 | +ve | 3.00-1500 | 0.993 |
| Average | | | | 0.988 |
| 15:0/18:1(d7) PA | 2.20 | -ve | 0.70-350 | 0.899 |
| 15:0/18:1(d7) PC | 1.50 | -ve | 16.00-8000 | 0.988 |
| 15:0/18:1(d7) PE | 1.60 | -ve | 0.50-250 | 0.964 |
| 15:0/18:1(d7) PG | 1.20 | -ve | 3.0-1500 | 0.984 |
| 15:0/18:1(d7) PI | 2.40 | -ve | 1.00-500 | 0.985 |
| 15:0/18:1(d7) PS | 2.15 | -ve | 0.50-250 | - |
| Average | | | | 0.964 |

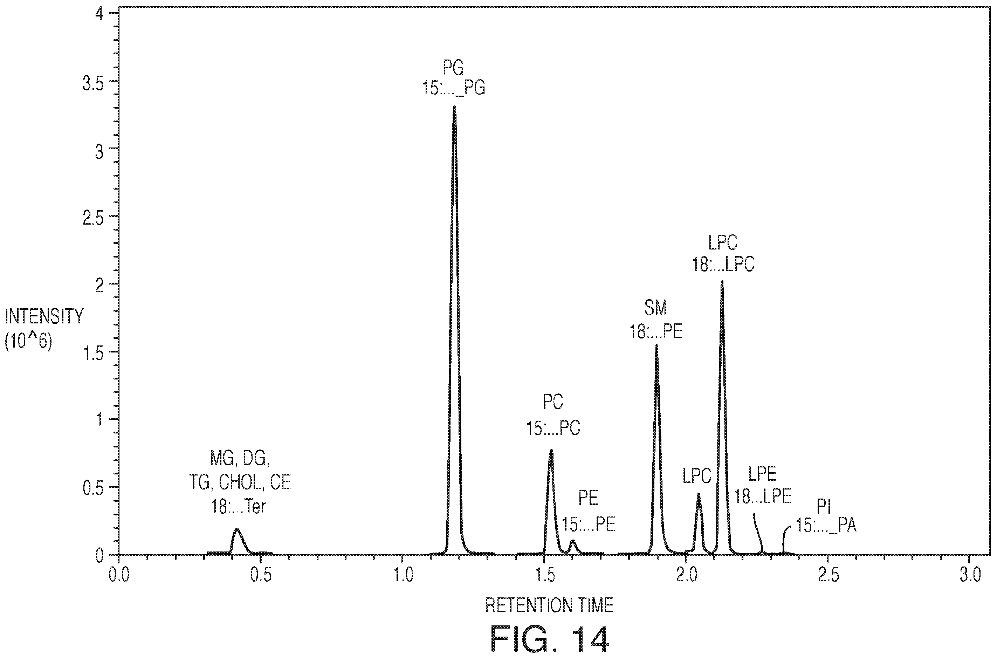

FIG. 14 is a positive ion mode chromatogram representing an example HILIC separation of a mixture of lipid standards, according to an embodiment of the present disclosure. As shown in FIG. 14, lipids are mainly separated according to their polarity into lipid classes within 8 min suitable for the lipidomic analysis of large sample sets. Retention time increases with the increase in lipid polarity.

The development of the library mentioned above allows for the simple download of all the MRM transitions and chromatographic conditions representing the different lipid classes with a total of 2041 MRM transitions, therefore eliminating manual input of LC-MS methods and reducing method development training cost and possible transcription errors.

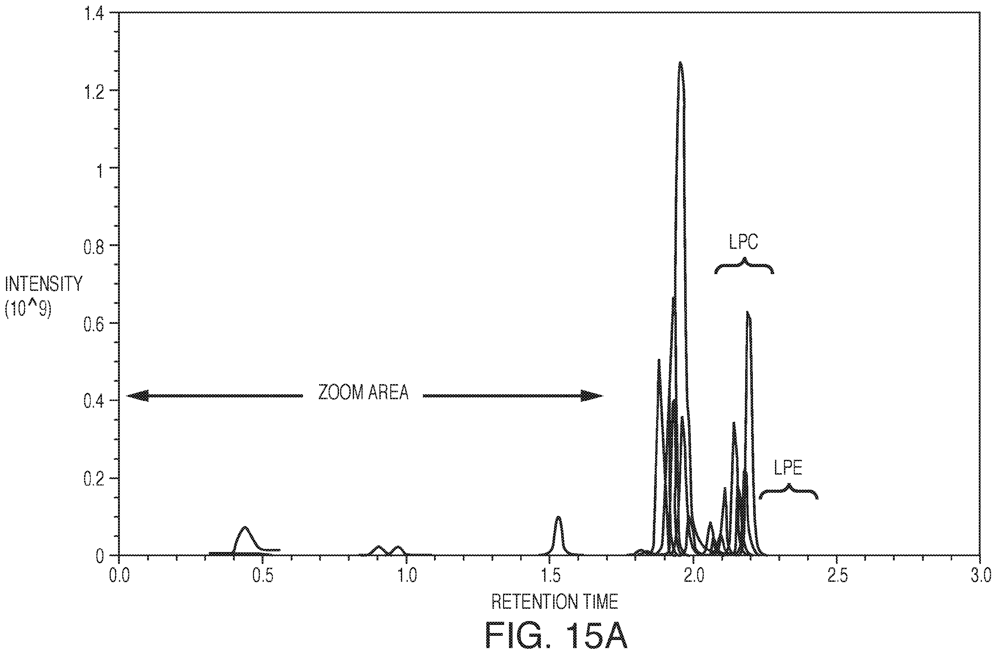

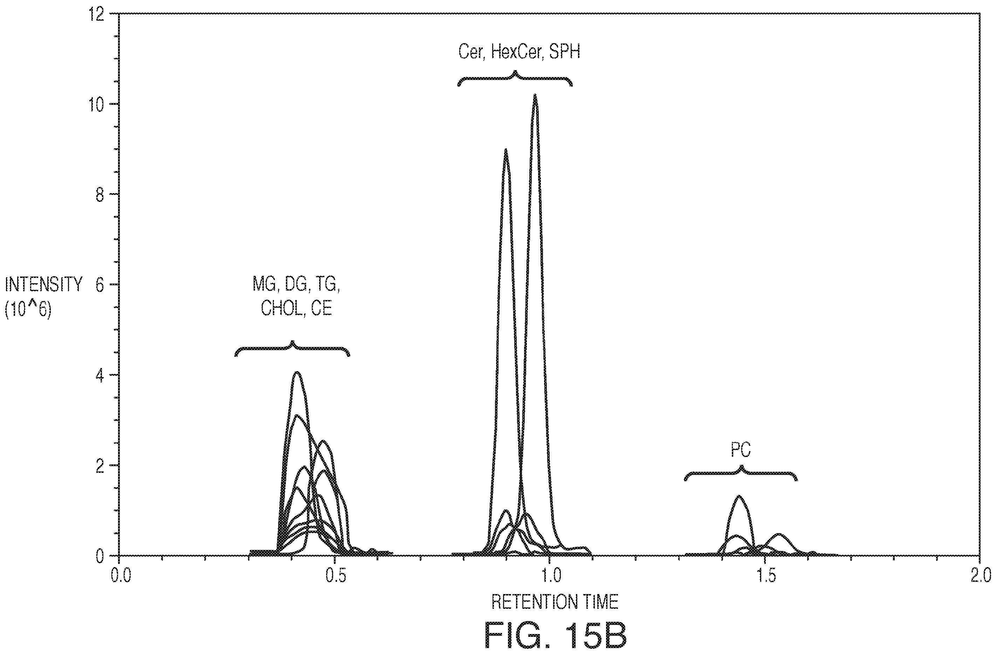

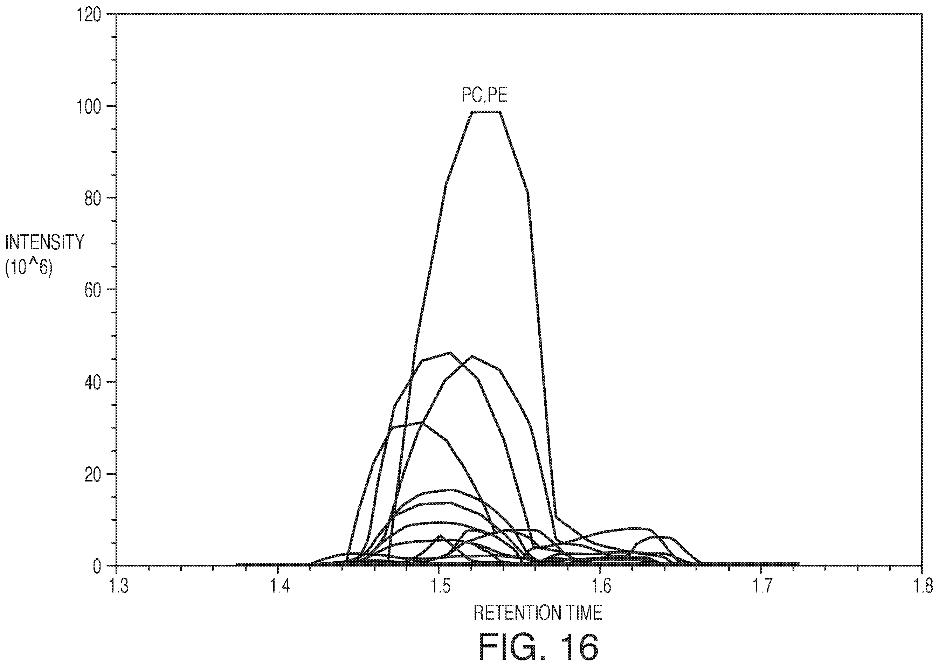

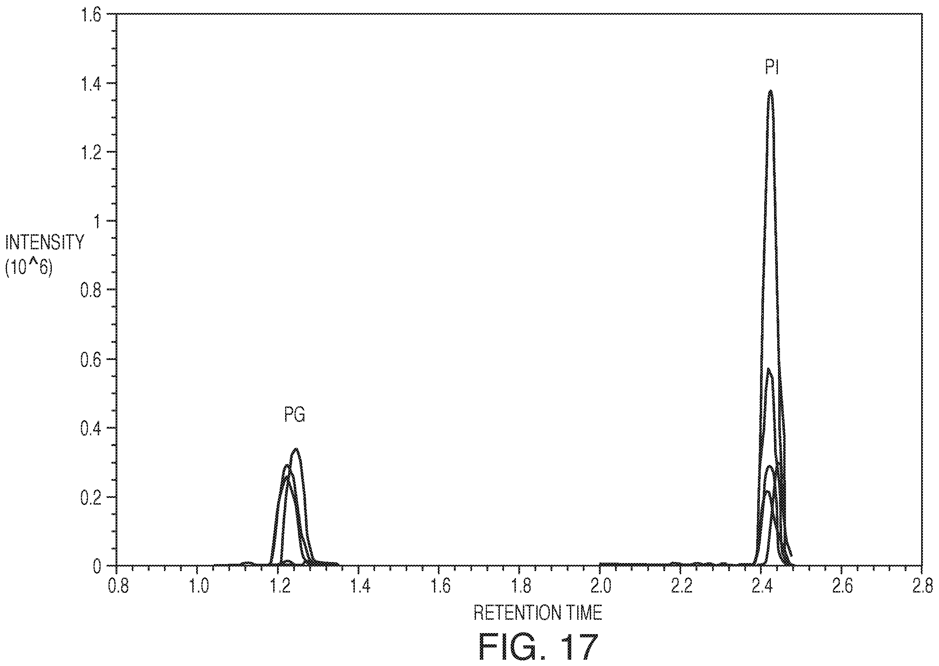

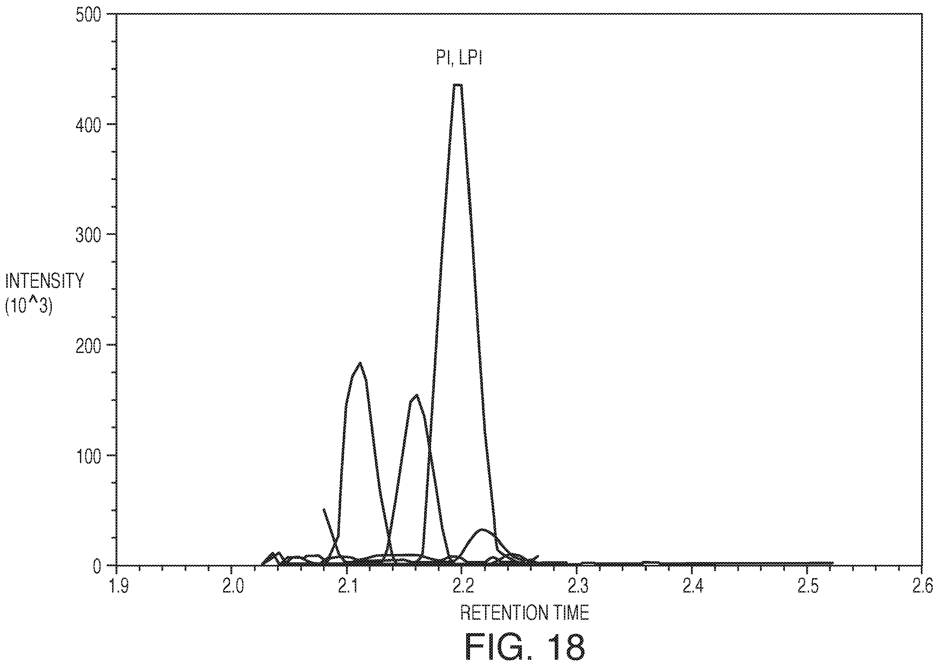

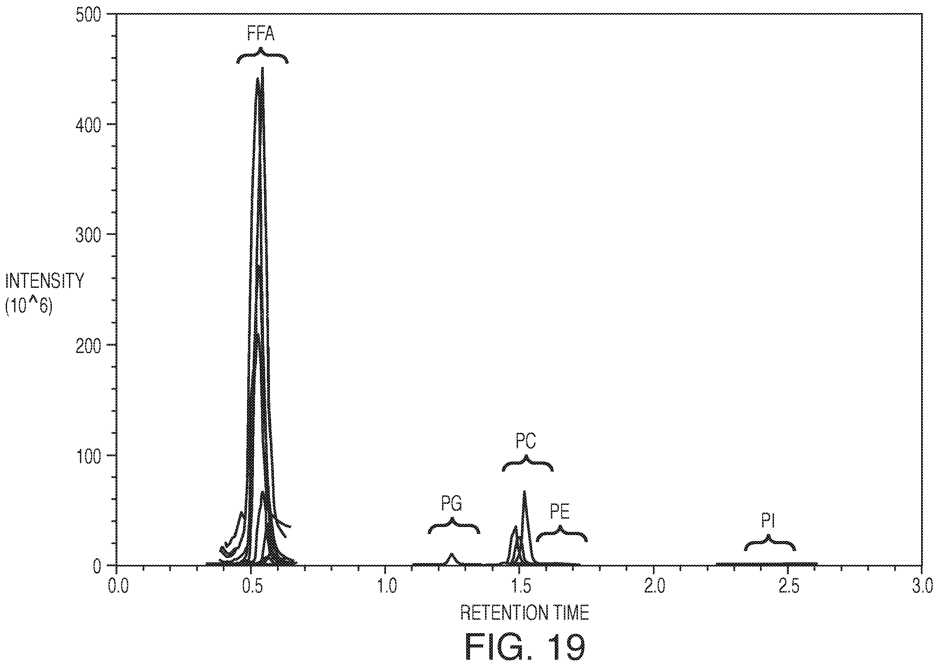

The developed method was used for the quantitative analysis of lipid species using SIL standards for each lipid class. Known concentration SIL standards were added to the plasma sample before extraction. FIG. 15 shows representative chromatogram HILIC lipid class separations in positive ion mode, according to an embodiment of the present disclosure. The inset chromatogram of FIG. 15 shows a zoom of the different lipid classes. FIGS. 16-19 show representative chromatogram HILIC lipid class separations in negative ion mode, according to an embodiment of the present disclosure. A total of 504 lipid species are identified and quantified from the NIST plasma (250 positive mode injection and 254 negative mode injection). The platform can also be used for more in depth targeting of specific class of lipids of interest. Table 1 shows calibration parameters of SIL standards spiked into the NIST plasma extract using positive and negative ion mode.

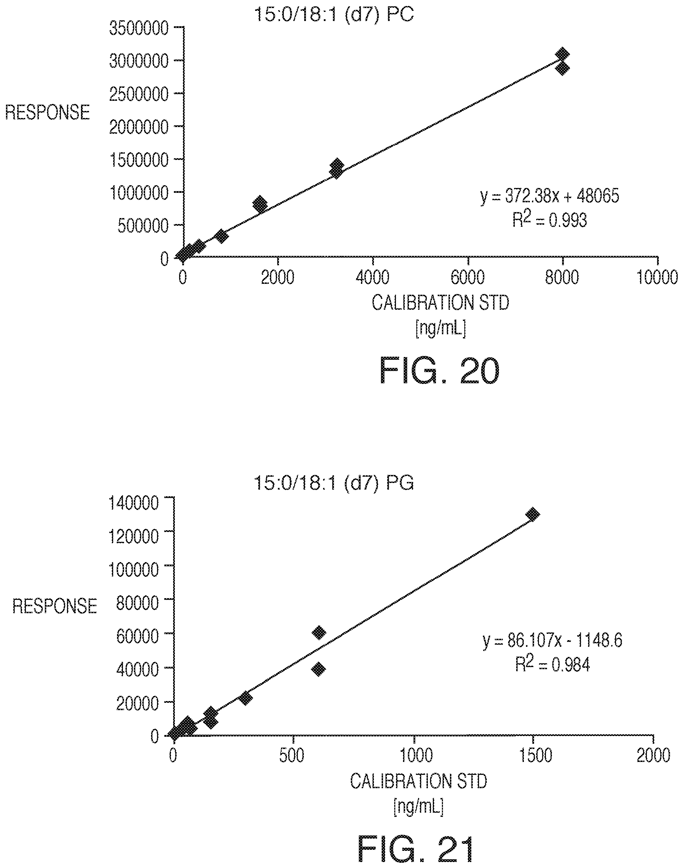

FIG. 20 shows a representative calibration curve for 15:0/18:1(d7) PC in positive ion mode, while FIG. 21 shows a representative calibration curve for 15:0/18:1(d7) PG in negative ion mode from a plasma sample, according to an embodiment of the present disclosure. Calibration curves were constructed for each SIL standards and are linear within tested calibration ranges with average correlation coefficients of greater than 0.988 (positive mode) and 0.964 (negative mode). Representative calibration curves for SIL standards 15:0/18:1(d7) PC in positive ion mode with 0.993 correlation coefficients and 15:0/18:1(d7) PG in negative ion mode with 0.985 correlation coefficients are shown in figures 20 and 21, respectively.

In this example embodiment, a high-throughput quantitative and comprehensive HILIC method was developed for the analysis of polar and non-polar lipid classes in plasma. The method enabled fast separation of 16 polar and non-polar lipid classes in 8 minutes suitable for the lipidomic analysis of large sample sets. The method was shown to be linear over 4-orders of magnitude and had sufficient sensitivity to allow for the analysis of lipids at systemic levels in human plasma.

### Example 4

Example 4 includes an example HILIC-based LC-MS/MS high-throughput targeted free fatty acid (FFA) screen. This example illustrates the rapid quantification of 24 FFAs in plasma and serum. As discussed above HILIC-based approach for the separation of lipids by class prior to MS analysis can reduce identification ambiguity. This example describes the use of a HILIC based approach to perform a targeted screen for FFA without the need for complex sample preparation and long chromatographic separation.

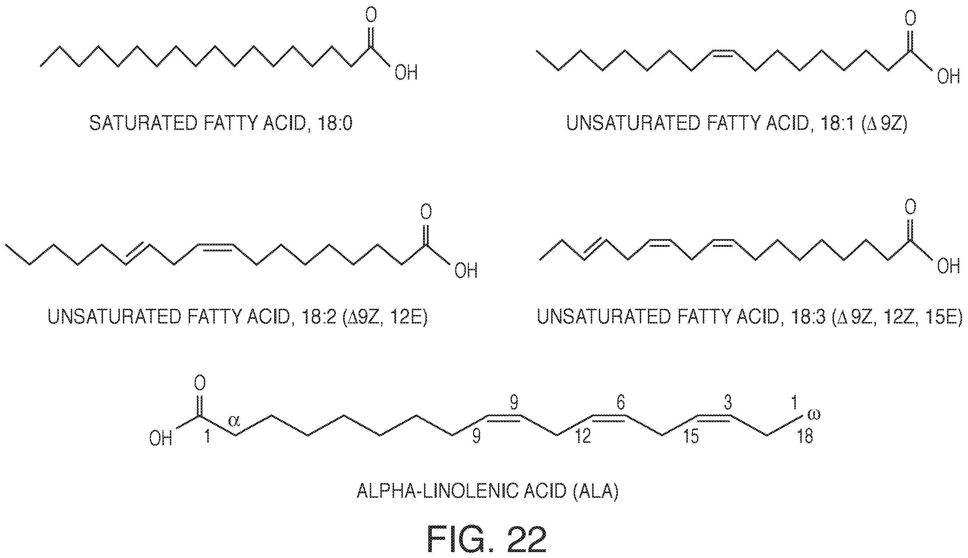

FIG. 22 shows the structure and nomenclature of different straight chain fatty acids. In this example experiment, pooled healthy control plasma was spiked with SIL (SPLASH LIPIDOMIX^{™}, Avanti Lipids, Alabaster, AL) at 9 concentration levels to generate calibration curves for quantification. SPLASH LIPIDOMIX^{™} does not contain a suitable surrogate standard for the quantification of FFA, but the linearity data can be used to assess the quality of the data generated. Typical R² values of 0.95 and deviations from the line of best fit (CVs <30%) are routinely achieved. PG (15:0-18: 1) (d7) = 0.5-1500 ng/mL and PC (15:0-18:1) (d7) = 16-8000 ng/mL. Six replicates of the NIST^{®} SRM^{®} 1950 plasma were also spiked with 5% SIL, prior to extraction.

A simple sample preparation procedure was adopted using protein precipitation with isopropanol (IPA) (1:5, plasma:IPA) and incubated for 2 hours. The extracted samples were centrifuged for 10 mins before transferring the supernatant to glass vials for LC-MS analysis.

The LC conditions for the analysis are described below in Table 14, and the MS conditions for the analysis are described below in Table 15.

**Table 14**

| | |
|---|---|
| LC System | ACQUITY I-class UPLC system (Fixed Loop (FL) or Flow Through Needle (FTN)) |
| Column(s) | Acquity UPLC BEH Amide 2.1 x100 mm, 1.7 µm |
| Column Temp. | 45 °C |
| Flow Rate | 0.6 mL/minute |
| Mobile Phase | 95:5 Acetonitrile / Water + 10 mM Ammonium Acetate (A) and 50:50 Acetonitrile / Water + 10 mM Ammonium Acetate (B) |
| Gradient | 0.1% to 20.0% B for 2 minutes, then 20% to 80% B for 3 minutes followed by 3 minutes re-equilibrium |
| Run Time | 8 Minutes |
| Injection Volume | 1 Microliter |

**Table 15**

| | |
|---|---|
| MS Systems | TQ-XS or TQ-S |
| Ionization Mode | ESI (-) |
| Capillary Voltage | 1.9 kV (-) |
| Acquisition Mode | MRM |
| Source Temperature | 120 °C |
| Desolvation Temperature | 500 °C |
| Cone Gas Flow | 150 L/hr |
| Desolvation Flow | 1000 L/hr |
| Nebuliser Gas | 7 bar |
| Ion Guide Offset 1 | 3 V |
| Ion Guide Offset 2 | 0.3 V |

In this example, a library was generated which contains the LC conditions, MS method, and associated processing method (including retention times). The resulting data were processed with either TargetLynx or Skyline (MacCoss Lab Software, University of Washington).

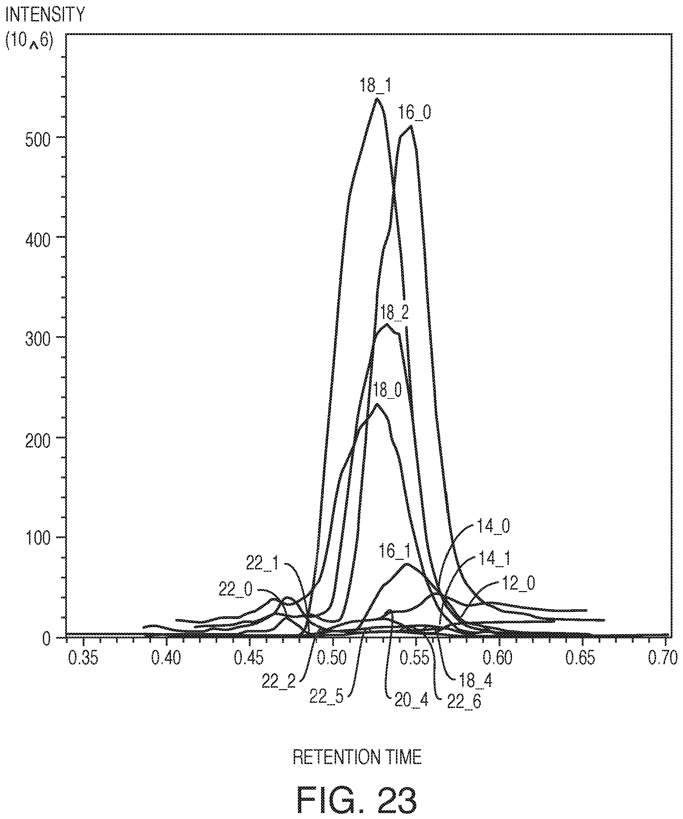

FIG. 23 shows an overlaid chromatogram representing a HILIC separation of endogenous FFA lipids in plasma, according to an embodiment of the present disclosure. Measurement of FFAs is typically performed using GC-MS. Prior to analysis, samples are typically fractionated using solid phases or liquid-liquid extraction techniques before hydrolysis with derivatisation agents to form fatty acid methyl esters (FAME). This protocol is time consuming, risks derivatising intact complex lipids and does not always perform well for the longer chain FAs (>C24) since these species are less volatile.

Alternatively, reversed-phase (RP) LC-MS can also be applied for the analysis of FFA, but this also requires time consuming sample preparation and the use of toxic organic solvents which are expensive to purchase and dispose of. Reversed-phase chromatography separates lipids according to chain-length and degree of unsaturation. The dual nature of reversed-phase separation (a double bond in the fatty acyl chain reduces retention time and the fatty acyl chain length increases retention time) can hamper the analysis of real samples; due to co-elution the number of components is often so great that identification becomes difficult.

This example method employs pseudo MRM in negative ion mode to identify and quantify 24 FFA species contained in human plasma while simultaneously analysing several other phospholipid classes from the same injection. FFAs elute as a discrete band (~0.5 mins) under HILIC based conditions, as can be seen in FIG. 23. The method sensitivity easily facilitated the detection of these lipids in human plasma at normal circulating levels from 50 µL of plasma, while demonstrating a linear dynamic range over 4 orders of magnitude.

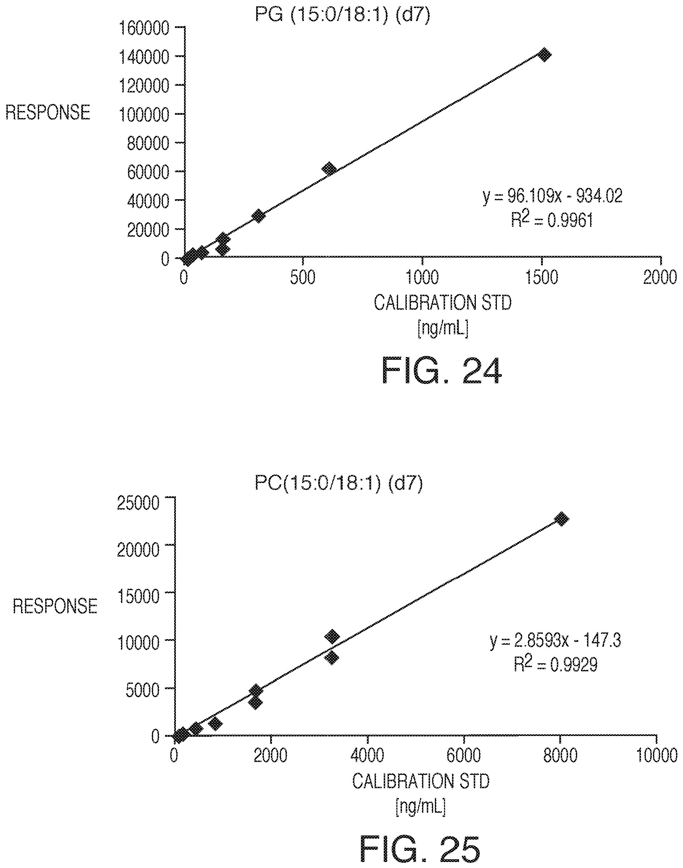

FIG. 24 shows a representative calibration curve for PG (15:0/18:1) (d7) (16-8000 ng/mL), according to an embodiment of the present disclosure. FIG. 25 shows a representative calibration curve for PC (15:0/18:1) (d7) (16-8000 ng/mL), according to an embodiment of the present disclosure. Table 16 shows the peak area response for lipid species with CVs <30% (17 out of 24). Typical R² values of 0.95 and deviations from the line of best fit (CVs <30%) are routinely achieved using the available SIL from SPLASH LIPIDOMIX^{™}, The calibration curve for PG (15:0-18:1) (d7) = 0.5-1500 ng/mL and PC (15:0-18:1) (d7) = 16-8000 ng/mL from the same analysis are shown in FIGS. 24 and 25, respectively.

**Table 16**

| Common Name | Method Name | MRM Transition | RT (mins) | Average Response | Std Dev | CV % |
|---|---|---|---|---|---|---|
| Lauric acid | FFA_12_0 | 199.2>199.2 | 0.58 | 429879 | 64433.8 | 15.0 |
| Myristic acid | FFA_14_0 | 227.2>227.2 | 0.56 | 1485548 | 161458.3 | 10.9 |
| Myristoleic acid | FFA_14_1 | 225.2>225.2 | 0.56 | 325245 | 39845.8 | 12.3 |
| Palmitic acid | FFA_16_0 | 255.2>255.2 | 0.55 | 17067070 | 1743697.1 | 10.2 |
| Palmitoleic acid | FFA_16_1 | 253.2>253.2 | 0.55 | 3675190 | 360805.3 | 9.82 |
| Margaric acid | FFA_17_0 | 269.2>269.2 | 0.54 | 355755 | 41596.8 | 11.7 |
| Stearic acid | FFA_18_0 | 283.3>283.3 | 0.53 | 7490717 | 1792103.0 | 23.9 |
| Oleic acid | FFA_18_1 | 281.2>281.2 | 0.53 | 22696457 | 2245943.6 | 9.90 |
| Linoleic acid | FFA_18_2 | 279.2>279.2 | 0.53 | 13103876 | 1323500.8 | 10.1 |
| Linolenic acid | FFA_18_3 | 277.2>277.2 | 0.54 | 1077326 | 116294.1 | 10.8 |
| Eicosadienoic acid | FFA_20_2 | 307.3>307.3 | 0.52 | 254423 | 29137.4 | 11.5 |
| Eicosatrienoic acid | FFA_20_3 | 305.2>305.2 | 0.52 | 333353 | 40288.7 | 12.1 |
| Arachidonic acid | FFA_20_4 | 303.2>303.2 | 0.53 | 1084491 | 133572.3 | 12.3 |
| Eicosapentaenoic acid | FFA_20_5 | 301.2>301.2 | 0.54 | 56543 | 6801.7 | 12.0 |
| Docosatrienoic acid | FFA_22_3 | 333.3>333.3 | 0.49 | 36426 | 6102.8 | 16.8 |
| Docosatetraenoic acid | FFA_22_4 | 331.3>331.3 | 0.49 | 158598 | 35290.4 | 22.3 |
| Docosapentaenoic acid | FFA_22_5 | 329.2>329.2 | 0.52 | 154336 | 28112.9 | 18.2 |

A hydrophilic interaction chromatography (HILIC) based approach avoids the need for derivatization and complex sample preparation associated with FAME GC-MS. Additionally, since lipids are separated by class, the risk of co-elution associated with RP LC-MS is also minimized. The development of the library discussed above allows for the simple download of MRM transitions and chromatographic conditions representing FFAs, therefore eliminating manual input of LC-MS methods and reducing possible transcription errors.

### Example 5

A sample preparation procedure was adopted using protein precipitation with pre-cooled isopropanol (IPA) (1:5, plasma:IPA). Samples were vortex mixed for one minute and placed at -20 °C for 10 minutes. Samples were vortex mixed again for one minute and placed at 4 °C for two hours to ensure complete protein precipitation. The extracted samples were centrifuged at 10,300 g for 10 minutes at 4 °C and the supernatant was transferred to glass vials for LC-MS/MS analysis. Prepared samples were analyzed in duplicate for both positive and negative ionization modes.

The LC conditions for the analysis are described below in Table 17, and the MS conditions for the analysis are described below in Table 18.

**Table 17**

| | |
|---|---|
| LC System | ACQUITY I-class UPLC system (Fixed Loop (FL) or Flow Through Needle (FTN)) |
| Column(s) | Acquity UPLC BEH Amide 2.1 x100 mm, 1.7 µm |
| Column Temp. | 45 °C |
| Flow Rate | 0.6 mL/minute |
| Mobile Phase | 95:5 Acetonitrile / Water + 10 mM Ammonium Acetate (A) and 50:50 Acetonitrile / Water + 10 mM Ammonium Acetate (B) |
| Gradient | 0.1% to 20.0% B for 2 minutes, then 20% to 80% B for 3 minutes followed by 3 minutes re-equilibrium |
| Run Time | 8 Minutes |
| Injection Volume | 2 Microliter |

**Table 18**

| MS Systems | TQ-XS or TQ-S micro |
|---|---|
| Ionization Mode | ESI (+/-) |
| Capillary Voltage | 2.8 kV (+) 1.9 kV (-) |
| Acquisition Mode | MRM |
| Source Temperature | 120 °C |
| Desolvation Temperature | 500 °C |
| Cone Gas Flow | 150 L/hr |
| Desolvation Flow | 1000 L/hr |
| Nebuliser Gas | 7 bar |
| Ion Guide Offset 1 | 3 V |
| Ion Guide Offset 2 | 0.3 V |

Plasma samples from three biological states of varying phenotype (healthy control, COPD patients, and asthma patients) were analyzed. Samples were prepared using a simple protein precipitation with cooled IPA before targeted LC-MS/MS data were acquired in positive and negative ion electrospray modes. Samples were randomized and two technical replicates per sample were acquired. A large number of lipids were identified from the HILIC-based quantification method MRM library containing highly specific fatty acyl transitions and head group fragments for increased identification confidence and increased specificity. An example of such a library can include the LipidQuan^{™} method file (Waters Technologies Corp. Milford, MA 01757).

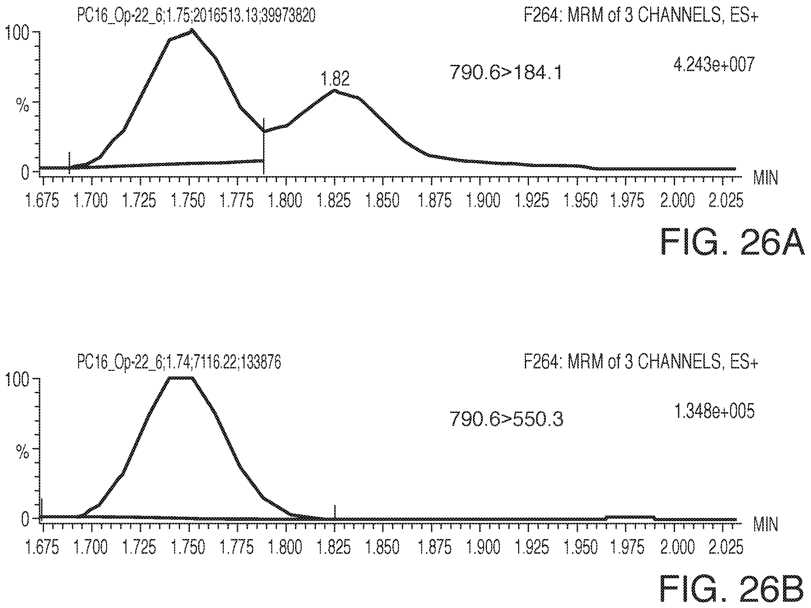

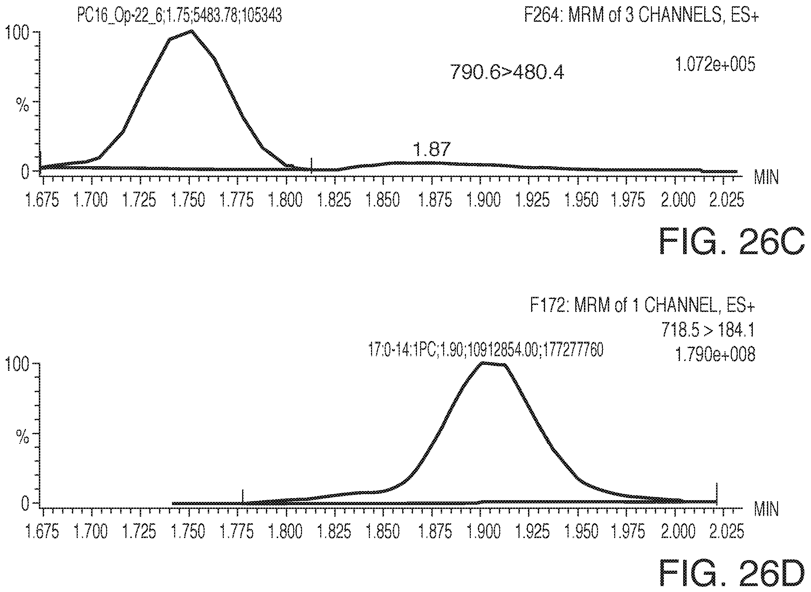

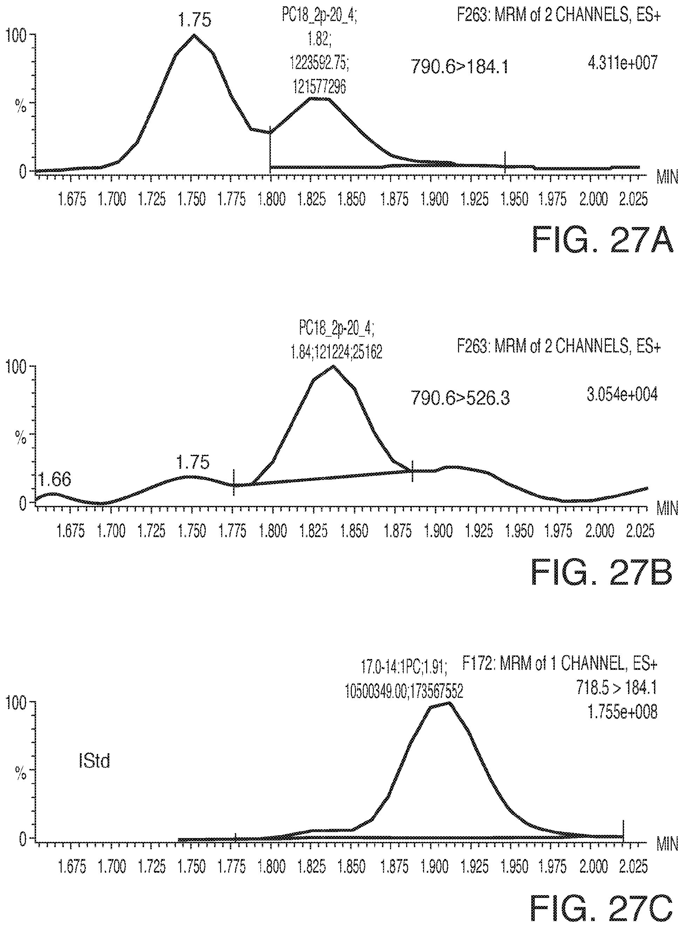

The power of the HILIC-based quantification method MRM library is illustrated in FIGS 26A-26D and FIGS. 27A-27C. In this example, phosphocholines (PC), which have two potential identifications from the same precursor mass, were distinguished by the specific fatty acyl chain fragments. In FIG. 26A and FIG. 26C, a choline head group can be identified at approximately 1.75 min. In FIG. 26B a choline head group can be identified at approximately 1.74 min, while in FIG. 26D a choline head group can be identified at approximately 1.90 min. In FIG. 27A, a choline head group can be identified at approximately 1.75 min; in FIG. 27B a choline head group can be identified at approximately 1.84 min; and in FIG. 27C a choline head group can be identified at approximately 1.91 min. In both FIG. 26A and 27A, a second choline head group can also be identified at approximately 1.82 min.

### Example 6

A sample preparation procedure was adopted using protein precipitation with pre-cooled isopropanol (IPA) (1:5, plasma:IPA). Samples were vortex mixed for one minute and placed at -20 °C for 10 minutes. Samples were vortex mixed again for one minute and placed at 4 °C for two hours to ensure complete protein precipitation. The extracted samples were centrifuged at 10,300 g for 10 minutes at 4 °C and the supernatant was transferred to glass vials for LC-MS/MS analysis. A difference between this example and the one described in Example 4, above, is that samples were analyzed for both positive and negative ionization modes. As illustrated below, this example was performed at a different injection volume than the one described in Example 5.

The LC conditions for the analysis are described below in Table 19, and the MS conditions for the analysis are described below in Table 20.

**Table 19**

| | |
|---|---|
| LC System | ACQUITY I-class UPLC system (Fixed Loop (FL) or Flow Through Needle (FTN)) |
| Column(s) | Acquity UPLC BEH Amide 2.1 x100 mm, 1.7 µm |
| Column Temp. | 45 °C |
| Flow Rate | 0.6 mL/minute |
| Mobile Phase | 95:5 Acetonitrile / Water + 10 mM Ammonium Acetate (A) and 50:50 Acetonitrile / Water + 10 mM Ammonium Acetate (B) |
| Gradient | 0.1% to 20.0% B for 2 minutes, then 20% to 80% B for 3 minutes followed by 3 minutes re-equilibrium |
| Run Time | 8 Minutes |
| Injection Volume | 1 Microliter |

**Table 20**

| MS Systems | TQ-XS or TQ-S |
|---|---|
| Ionization Mode | ESI (+/-) |
| Capillary Voltage | 2.8 kV (+) 1.9 kV (-) |
| Acquisition Mode | MRM |
| Source Temperature | 120 °C |
| Desolvation Temperature | 500 °C |
| Cone Gas Flow | 150 L/hr |
| Desolvation Flow | 1000 L/hr |
| Nebuliser Gas | 7 bar |
| Ion Guide Offset 1 | 3 V |
| Ion Guide Offset 2 | 0.3 V |

In this example, a rapid and comprehensive LC-MS/MS method was developed for the analysis of polar and non-polar lipid classes in human plasma employing a HILIC-based lipid class separation and MRM MS quantitation in both positive and negative ion modes. Table 21 shows the list of SIL standards spiked into the NIST 1950 plasma extract and the calibration parameters in positive and negative ion mode.

**Table 21**

| **SIL standard** | **Retention time (min)** | **Acquisition mode** | **Calibration Range (ng/mL)** | **Correlation Coefficient** |
|---|---|---|---|---|
| 15:0/18:1(d7)/15:0 TG | 0.40 | +ve | 5.50-2750 | 0.994 |
| 18:1(d7) LPC | 2.08 | +ve | 2.50-1250 | 0.9936 |
| 18:1(d7) LPE | 2.27 | +ve | 0.50-250 | 0.9877 |
| 15:0/18:1(d7) PC | 1.50 | +ve | 16.00-8000 | 0.9934 |
| 18:1/18:1(d9) SM | 1.88 | +ve | 3.00-1500 | 0.9933 |
| **Average positive mode** | | | | **0.9924** |
| 15:0/18:1(d7) PC | 1.50 | -ve | 16.00-8000 | 0.9882 |
| 15:0/18:1(d7) PE | 1.60 | -ve | 0.50-250 | 0.9644 |
| 15:0/18:1(d7) PG | 1.20 | -ve | 3.0-1500 | 0.9840 |
| 15:0/18:1(d7) PI | 2.40 | -ve | 1.00-500 | 0.9845 |
| **Average negative mode** | | | | **0.9803** |

Table 22 below shows the different lipid classes measured with their corresponding MRM retention time window, acquisition mode used, and the total number of MRM transitions, according to this example embodiment. Lipids are mainly separated into lipid classes according to their polarity within about 8 minutes, yielding a quantitative method suitable for the lipidomic analysis of large sample sets. Retention time increases with the increase in lipid polarity, in this example embodiment.

**Table 22**

| **Lipid Class** | **Retention time (min)** | **Acquisition mode** | **Number of MRM transitions** | **Number of Lipid Species** |
|---|---|---|---|---|
| Cholesterol ester | 0.42 | +ve | 10 | 10 |
| MG | 0.50 | +ve | 7 | 7 |
| DG | 0.51 | +ve | 73 | 39 |
| TG | 0.41 | +ve | 38 | 20 |
| Ceramide | 0.90 | +ve | 24 | 24 |
| Hexosyl Ceramide | 0.90 | +ve | 23 | 23 |
| LPC | 2.08 | +ve | 22 | 22 |
| LPE | 2.25 | +ve | 24 | 24 |
| PC | 1.50 | +ve | 175 | 59 |
| SM | 1.85 | +ve | 24 | 24 |
| **Total positive mode** | | | **429** | **261** |
| SIL | | -ve | 6 | 6 |
| FFA | 0.51 | -ve | 27 | 27 |
| LPA | 2.80 | -ve | 5 | 5 |
| LPI | 3.00 | -ve | 6 | 6 |
| PA | 2.20 | -ve | 36 | 20 |
| PC | 1.50 | -ve | 123 | 64 |
| PE | 1.60 | -ve | 92 | 49 |
| PG | 1.20 | -ve | 35 | 20 |
| PI | 2.4 | -ve | 59 | 32 |
| PS | 2.15 | -ve | 30 | 18 |
| **Total negative mode** | | | 419 | **247** |

According to this example, the development of a HILIC-based quantification method file allows for the simple download of all the MRM transitions and chromatographic conditions representing the different lipid classes with a total of 2,041 MRM transitions, therefore eliminating manual input of LC-MS methods and reducing method development training costs and possible transcription errors. The developed method was used for the quantitative analysis of lipid species in NIST^{®} SRM^{®} 1950 plasma samples using SIL standards for each lipid class. Known concentration SIL standards were added to the NIST plasma sample before extraction. Table 21 above shows calibration parameters of SIL standards spiked into the NIST plasma extract using positive and negative ion mode. Calibration curves were constructed for each SIL standard, and are linear within tested calibration ranges with average correlation coefficients of greater than 0.992 (positive mode) and 0.980 (negative mode). These correlation coefficients demonstrate advantages of this new method.

### Example 7

Glycerolphospholipids (GPLs) generally consist of two fatty acyl chains and a phosphate head group esterified to a glycerol backbone, with the head group defining GPL. Lysophospholipids are derivatives of phospholipids where one or both of the fatty acyl chains have been removed. Biologically, GPLs are compounds of all biological membranes that act as a protective barrier with selective permeability characteristics to enable cellular metabolism. In recent years, research interest in measuring these lipid species has increased since they are known to be involved in numerous diseases. PE is the second most abundant gycerolphospholipid (after phosphatidylcholines) and includes between about 15-25% of the total GPL content in mammalian cells. Disturbances in PE metabolism have been implicated in both chronic (Alzheimer's and Parkinson's) and infectious (Candidiasis) diseases. Only a small component of PG is observed in eukaryotic mitochondrial membranes. However, PG is the biosynthetic precursor of cardiolipin, which represents a major constituent of mitochondria membrane and is important in maintaining the potential of these membranes. PI plays a small role in membrane structure but plays a major role in membrane-bound signaling processes and vesicular activity.

Although advances in MS have allowed for more in depth lipodomic analysis, unambiguous identification and quantification has proven difficult as lipids have a high number of isomeric and isobaric lipid species. MS spectra often contain multiple peaks and fragments from various compounds making confident identification and relative quantitation of specific molecular species difficult or challenging. As a result, lipidomic data generated during multi-site studies may not be commutative and resulting biological interpretation of the data questionable. This example describes the use of the HILIC-based quantification method discussed herein to perform a targeted screen for LPEs, PEs, PGs, and PIs.

The LC conditions for the analysis are described below in Table 23, and the MS conditions for the analysis are described below in Table 24.

**Table 23**

| | |
|---|---|
| LC System | ACQUITY I-class UPLC system (Fixed Loop (FL) or Flow Through Needle (FTN)) |
| Column(s) | Acquity UPLC BEH Amide 2.1 x100 mm, 1.7 µm |
| Column Temp. | 45 °C |
| Flow Rate | 0.6 mL/minute |
| Mobile Phase | 95:5 Acetonitrile / Water + 10 mM Ammonium Acetate (A) and 50:50 Acetonitrile / Water + 10 mM Ammonium Acetate (B) |
| Gradient | 0.1% to 20.0% B for 2 minutes, then 20% to 80% B for 3 minutes followed by 3 minutes re-equilibrium |
| Run Time | 8 Minutes |
| Injection Volume | 2 Microliter (-ve mode) 1 Microliter (+ve mode) |

**Table 24**

| MS Systems | TQ-XS, TQ-S, or TQ-S micro |
|---|---|
| Ionization Mode | ESI (+/-) |
| Capillary Voltage | 2.8 kV (+) 1.9 kV (-) |
| Acquisition Mode | MRM |
| Source Temperature | 120 °C |
| Desolvation Temperature | 500 °C |
| Cone Gas Flow | 150 L/hr |
| Desolvation Flow | 1000 L/hr |
| Nebuliser Gas | 7 bar |
| Ion Guide Offset 1 | 3 V |
| Ion Guide Offset 2 | 0.3 V |

In this example, a rapid and comprehensive LC-MS/MS method was developed for the analysis of LPE, PE, PG, and PI lipids in human plasma employing a HILIC-based chromatographic separation and MRM MS detection. PEs, PG, and PI were analyzed in negative ion mode, while LPE lipids were analyzed in positive ion mode, in this example. The HILIC methodology facilitated the elution of lipids in discrete classes with the PGs eluting first (~1.21 mins) followed by PEs (~1.62 mins), LPEs (~2.34 mins), and PIs (~2.40 mins). Using this method, 11 LPEs, 47 PEs, 21 PGs, and 33 PIs were quantitatively identified in eight minutes with a linear dynamic range over four orders of magnitude. The method sensitivity easily facilitated the detection of these lipids in human plasma at normal circulating levels from 50 uL of plasma.

The development of a HILIC-based quantification method file allows for the simple download and importing of MRM transitions and chromatographic conditions for LPEs, PEs, PGs, and PIs and eliminates manual input of LC-MS/MS methods reducing possible transcription errors. The method file features highly specific MRM transitions for fatty acyl chain fragments contained in 219 PGs, 279 PEs, and 90 PIs enhancing the specificity of the method and improving lipid identification. Although LPE and PE species share a common head group, the method chromatographically resolves these lipids on the basis of class, thereby reducing potential isomeric and isobaric interferences. Isobaric effects are further minimized as the mass ranges of LPE and PE precursors, 398-476 Da and 686-851 Da, respectively, do not overlap.

Quantification was achieved, in this example, using calibration curves of plasma spiked with known concentrations of SIL standards prior to extraction. These SILs function as surrogate standards for the quantification of endogenous lipids within the same class. By using one surrogate standards per endogenous lipid class, rather than a SIL standard for each measured endogenous lipid, the cost of large studies can be significantly reduced.

Table 25 shows the back calculated concentrations of spiked SIL, as well as the actual concentration of SILs spiked into the NIST plasma in ng/mL. Table 26, below shows the MRM transitions of endogenous LPE lipids in NIST^{®} SRM^{®} 1950 plasma with CVs <30%. The calibration range of LPE (18:1) (d7) was 0.5-250 ng/mL, in this example.

**Table 25**

| **Calibration Range 2.5-1250 ng/mL** | RT [mins] | **MRM Transition** | Ave. Conc. [ng/mL] | Calc. Conc. [n=12] | Std Dev | CV [%] |
|---|---|---|---|---|---|---|
| LPE (18:1) (d7) | 2.34 | **487.4>346.2** | 250.0 | 256.1 | 27.0 | 10.5 |
| PI (15:0-18:1) (d7) | 2.40 | **828.6>288.3** | 500.0 | 490.9 | 93.7 | 19.1 |
| PE (15:0-18:1) (d7) | 1.62 | **709.5>288.3** | 250.0 | 206.5 | 30.3 | 14.7 |
| PG (15:0-18:1) (d7) | 1.21 | **740.6>288.3** | 1500.0 | 1205.5 | 166.9 | 13.8 |

**Table 26**

| **Lipid Class** | **Retention time (min)** | **MRM Transition** |
|---|---|---|
| LPE 16_0 | 2.37 | 454.3>313.2 |
| LPE 16_1 | 2.39 | 452.3>311.2 |
| LPE 17_1 | 2.36 | 466.3>325.2 |
| LPE 18_0 | 2.32 | 482.3>341.2 |
| LPE 18_1 | 2.34 | 480.3>339.2 |
| LPE 18_2 | 2.31 | 478.3>337.2 |

Table 27 shows MRM transitions of endogenous PE lipids, PI lipids, and PG lipids in this example embodiment. The calibration range of PE (15:0-18:1) (d7) was 0.5-250 ng/mL; the calibration range of PI (15:0-18: 1) (d7) was 1-500 ng/mL; and the calibration range of PG (15:0-18: 1) (d7) was 3-1500 ng/mL.

**Table 27**

| **Lipid Class** | **Retention time (min)** | **MRM Transition** |
|---|---|---|
| **PE** | | |
| PE_14_0-22_5 | 1.58 | 736.5>227.3 |
| | | 736.5>329.2 |
| PE_16_0-16_1 | 1.62 | 688.5>253.3 |
| | | 688.5>255.3 |
| PE (34:1) PE_16_0-18_1 | 1.61 | 716.7>255.3 |
| | | 716.5>281.3 |
| PE (34:1) PE_16_1-18_0 | 1.61 | 716.5>253.3 |
| | | 716.5>283.3 |
| PE (34:2) PE_16_0-18_2 | 1.61 | 714.5>255.3 |
| | | 714.5>279.3 |
| PE (34:2) PE_16_1-18_1 | 1.61 | 714.5>253.3 |
| | | 714.5>281.3 |
| PE_16_0-20_2 | 1.59 | 742.5>255.3 |
| | | 742.5>307.2 |
| PE_16_0-20_3 | 1.57 | 740.5>255.3 |
| | | 740.5>305.2 |
| PE_16_0-20_4 | 1.57 | 738.5>255.3 |
| | | 738.5>302.2 |
| PE_16_0-20_5 | 1.58 | 736.5>255.3 |
| | | 736.5>301.2 |
| PE_16_0-22_4 | 1.56 | 766.5>255.3 |
| | | 766.5>331.2 |
| PE_16_0-22_5 | 1.56 | 764.5>255.3 |
| | | 764.5>329.2 |
| PE_16_0-22_6 | 1.56 | 762.5>255.3 |
| | | 762.5>327.2 |
| PE_18-0-18_0 | 1.58 | 746.6>283.3 |
| PE_18-0-18_1 | 1.58 | 744.6>281.3 |
| | | 744.6>283.3 |
| PE_18_0-18_2 | 1.59 | 742.5>279.3 |
| | | 742.5>283.3 |
| PE_18_0-18_3 | 1.59 | 740.5>277.3 |
| | | 740.5>283.3 |
| PE_18_0-20_3 | 1.56 | 768.6>283.3 |
| | | 768.6>305.2 |
| PE_18_0-20-4 | 1.55 | 766.5>283.3 |
| | | 766.5>303.2 |
| PE_18_0-20_5 | 1.56 | 764.5>283.3 |
| | | 764.5>301.2 |
| PE_18_0-22_4 | 1.54 | 794.6>283.3 |
| | | 794.6>331.2 |
| PE_18-0-22_5 | 1.54 | 792.6>283.3 |
| | | 792.6>329.2 |
| PE_18_0-22_6 | 1.54 | 790.5>283.3 |
| | | 790.5>327.2 |
| PE_18_1-18_1 | 1.58 | 742.5>281.3 |
| PE_18_1-18_2 | 1.59 | 740.5>279.3 |
| | | 740.5>281.3 |
| PE_18_1-18_3 | 1.59 | 738.5>277.3 |
| | | 738.5>281.3 |
| PE_18_2-18_2 | 1.60 | 738.5>279.3 |
| PE_18_1-20_4 | 1.55 | 764.5>281.3 |
| | | 764.5>303.2 |
| PE_18_2-20_4 | 1.57 | 762.5>279.3 |
| | | 762.5>303.2 |

| **PI** | | |
|---|---|---|
| PI_16_0-16-0 | 2.39 | |
| | | 809.5>255.3 |
| PI_16-0-16_1 | 2.35 | 807.5>253.3 |
| | | 807.5>255.3 |
| PI (34:1) PI_16_0-18_1 | 2.33 | 835.5>255.3 |
| | | 835.5>281.3 |
| PE (34:1) PI_16_1-18_0 | 2.36 | 835.5>253.3 |
| | | 835.5>283.3 |
| PI (34:2) PI_16_0-18_2 | 2.34 | 833.5>355.3 |
| | | 833.5>279.3 |
| PI (34:2) PI_16_1-18_1 | 2.41 | 833.5>253.3 |
| | | 833.5>281.3 |
| PI_16_0-20_3 | 2.37 | 859.5>255.3 |
| | | 859.5>277.3 |
| PI_16_0-20_4 | 2.36 | 857.5>255.3 |
| | | 857.5>302.2 |
| PI_18-0-18_0 | 2.42 | 865.6>283.3 |
| PI_18_0-18_1 | 2.36 | 863.6>283.3 |
| | | 863.6>281.3 |
| PI_18_0-18_2 | 2.31 | 861.5>283.3 |
| | | 861.5>279.3 |
| PI_18_0-20_2 | 2.34 | 889.6>283.3 |
| | | 889.5>307.2 |
| PI_18_0-20_3 | 2.39 | 887.6>283.3 |
| | | 887.6>305.2 |
| PI_18_0-20_4 | 2.39 | 885.5>283.3 |
| | | 885.5>302.2 |
| PI_18_1-18_1 | 2.36 | 861.5>281.3 |
| PI_18_1-18_2 | 2.36 | 859.5>279.3 |
| | | 859.5>281.3 |
| PI_18-2-18_2 | 2.39 | 857.5>279.3 |

| **PG** | | |
|---|---|---|
| PG_16_0-18_1 | 1.21 | 747.5>255.3 |
| | | 747.5>281.3 |
| PG_16_0-18_2 | 1.20 | 745.5>255.3 |
| | | 745.5>279.3 |
| PG_18_0-18_1 | 1.19 | 775.5>281.3 |
| | | 775.5>283.3 |
| PG_18_0-18_2 | 1.19 | 773.5>279.3 |
| | | 773.5>283.3 |

As demonstrated in this example, the use of the HILIC-based quantification method discussed herein provides the advantage of performing targeted screens for LPE lipids, PE lipids, PG lipids, and PI lipids.

### Example 8

In this example, a rapid, specific LC-MS/MS method was developed for the analysis of PC, LPC, and SM lipids in human plasma employing a HILIC-based chromatographic separation and MRM MS detection in both positive and negative ion mode. The HILIC methodology facilitated the elution of lipids in discrete classes with the PCs eluting first (~1.52 mins) followed by SMs (~1.92 mins), and LPCs (~2.15 mins). The resulting method was capable of measuring 61 PCs, 21 LPCs, and 24 SMs in eight minutes with a linear dynamic range over four orders of magnitude. The method sensitivity easily facilitated the detection of these lipids in human plasma at normal circulating levels from 50 uL of plasma. Product ion scans of these three lipid classes showed the characteristic 184 m/z choline head group fragment in positive ion mode, that results in unambiguous lipid assignment if employed in an MRM transition. To improve identification and specificity, highly specific MRM transitions containing fatty acyl chain fragments for 106 PCs (positive ion) and 278 PCs (negative ion) were developed. Although the MRM transitions for LPC and SM species employ the choline head group fragment, chromatographic separation on the basis of class reduces isomeric and isobaric interferences. Additionally, isobaric effects can be further minimized since LPC and SM precursors have mass ranges of 440-608 Da and 648-776 Da, respectively, and therefore do not overlap.

The LC conditions for the analysis are described below in Table 28, and the MS conditions for the analysis are described below in Table 29.

**Table 28**

| LC System | ACQUITY I-class UPLC system (Fixed Loop (FL) or Flow Through Needle (FTN)) |
|---|---|
| Column(s) | Acquity UPLC BEH Amide 2.1 x100 mm, 1.7 µm |
| Column Temp. | 45 °C |
| Flow Rate | 0.6 mL/minute |
| Mobile Phase | 95:5 Acetonitrile / Water + 10 mM Ammonium Acetate (A) and 50:50 Acetonitrile / Water + 10 mM Ammonium Acetate (B) |
| Gradient | 0.1% to 20.0% B for 2 minutes, then 20% to 80% B for 3 minutes followed by 3 minutes re-equilibrium |
| Run Time | 8 Minutes |
| Injection Volume | 1 Microliter |

**Table 29**

| MS Systems | TQ-XS, TQ-S, or TQ-S micro |
|---|---|
| Ionization Mode | ESI (+/-) |
| Capillary Voltage | 2.8 kV (+) 1.9 kV (-) |
| Acquisition Mode | MRM |
| Source Temperature | 120 °C |
| Desolvation Temperature | 500 °C |
| Cone Gas Flow | 150 L/hr |
| Desolvation Flow | 1000 L/hr |
| Nebuliser Gas | 7 bar |
| Ion Guide Offset 1 | 3 V |
| Ion Guide Offset 2 | 0.3 V |

Table 30 shows MRM transitions of endogenous LPC lipids, Table 31 shows the MRM transitions of PC lipids, and Table 32 shows the MRM transitions of SM lipids in this example embodiment. The calibration range of LPC (18:1) (d7) was 2.5-1250 ng/mL; the calibration range of PC (15:0-18:1) (d7) was 16-8000 ng/mL; and the calibration range of SM (18:1) (d7) was 3-1500 ng/mL.

**Table 30**

| Lysophosphatidylcholine (LPC) | RT (mins) | MRM transition |
|---|---|---|
| LPC 15_0 | 2.22 | 482.3>184.1 |
| LPC 16_0 | 2.20 | 496.3>184.1 |
| LPC 16_1 | 2.21 | 494.3>184.1 |
| LPC 18_0 | 2.15 | 524.4>184.1 |
| LPC 18_1 | 2.16 | 522.4>184.1 |
| LPC 18_2 | 2.18 | 520.3>184.1 |
| LPC 18_3 | 2.20 | 518.3>184.1 |
| LPC 20_0 | 2.10 | 552.4>184.1 |
| LPC 20_3 | 2.14 | 546.4>184.1 |
| LPC 20_4 | 2.14 | 544.3>184.1 |
| LPC 20_5 | 2.16 | 542.3>184.1 |
| LPC 22_1 | 2.18 | 578.4>184.1 |
| LPC 22_4 | 2.11 | 572.3>184.1 |
| LPC 22_6 | 2.13 | 568.3>184.1 |
| LPC 24_0 | 2.03 | 608.5>184.1 |
| LPC O- 16_0 | 2.22 | 482.4>184.1 |
| LPC O- 18_0 | 2.17 | 510.4>184.1 |
| LPC P-18_1 | 2.04 | 506.4>184.1 |
| LPC P-20_0 | 2.14 | 536.4>184.1 |

**Table 31**

| **Phosphatidycholine (PC)** | **RT (mins)** | **MRM transition** |
|---|---|---|
| PC 14_0-16_0 | 1.56 | 764.5>227.3 |
| | | 764.5>255.3 |
| PC 16_0-16_0 | 1.54 | 792.6>255.3 |
| PC 16_0-16_1 | 1.54 | 790.6>253.3 |
| | | 790.6>255.3 |
| PC 16_0-18_0 | 1.50 | 820.6>255.3 |
| | | 820.6>283.3 |
| PC (34:1) PC 16_0-18_1 | 1.50 | 818.6>255.3 |
| | | 818.6>281.3 |
| PC (34:1) PC 16_1-18_0 | 1.50 | 818.6>253.3 |
| | | 818.6>283.3 |
| PC (34:2) PC 16_0-18_2 | 1.51 | 816.6>255.3 |
| | | 816.6>279.3 |
| PC (34:2) PC 16_1-18_1 | 1.50 | 816.6>253.3 |
| | | 816.6>281.3 |
| PC (34:3) PC 16_0-18_3 | 1.51 | 814.6>255.3 |
| | | 814.6>277.3 |
| PC (34:3) PC 16_1-18_2 | 1.51 | 814.6>253.3 |
| | | 814.6>279.3 |
| PC (36:1) PC 16_0-20_1 | 1.49 | 846.6>255.3 |
| | | 814.6>309.2 |
| PC (36:1) PC 18_0-18_1 | 1.48 | 846.6>281.3 |
| | | 846.6>283.3 |
| PC (36:2) PC 16_0-20_2 | 1.49 | 844.6>255.3 |
| | | 844.6>307.2 |
| PC (36:2) PC 18_0-18_2 | 1.49 | 844.6>279.3 |
| | | 844.6>283.3 |
| PC (36:2) PC 18_1-18_1 | 1.48 | 844.6>281.3 |
| PC (36:3) PC 16_0-20_3 | 1.48 | 842.6>255.3 |
| | | 842.6>305.2 |
| PC (36:3) PC 18_0-18_3 | 1.48 | 842.6>277.3 |
| | | 842.6>283.3 |
| PC (36:3) PC 18_1-18_2 | 1.49 | 842.6>279.3 |
| | | 842.6>281.3 |
| PC (36:4) PC 16_0-20_4 | 1.47 | 840.6>255.3 |
| | | 840.6>303.2 |
| PC (36:4) PC 16_1-20_3 | 1.48 | 840.6>253.3 |
| | | 840.6>305.3 |
| PC (36:4) PC 18_1-18_3 | 1.49 | 840.6>277.3 |
| | | 840.6>281.3 |
| PC (36:4) PC 18_2-18_2 | 1.49 | 840.6>279.3 |
| PC (36:5) PC 16_0-20_5 | 1.47 | 838.6>255.3 |
| | | 838.6>301.2 |
| PC (36:5) PC 16_1-20_4 | 1.47 | 838.6>253.3 |
| | | 838.6>303.2 |
| PC 18_1-20_0 | 1.45 | 874.7>281.3 |
| | | 874.7>311.2 |
| PC (38:2) PC 18_0-20_2 | 1.47 | 872.6>283.3 |
| | | 872.6>307.2 |
| PC (38:2) PC 18_1-20_1 | 1.47 | 872.6>281.3 |
| | | 872.6>309.2 |
| PC (38:2) PC 18_2-20_0 | 1.46 | 872.6>279.3 |
| | | 872.6>311.2 |
| PC (38:3) PC 16_0-22_3 | 1.46 | 870.6>255.3 |
| | | 870.6>333.2 |
| PC (38:3) PC 18_1-20_2 | 1.47 | 870.6>281.3 |
| | | 870.6>307.2 |
| PC (38:3) PC 18_0-20_3 | 1.46 | 870.6>283.3 |
| | | 870.6>305.2 |
| PC (38:4) PC 16_0-22_4 | 1.47 | 868.6>255.3 |
| | | 868.6>331.2 |
| PC (38:4) PC 18_2-20_2 | 1.48 | 868.6>279.3 |
| | | 868.6>307.2 |
| PC (38:5) PC 16_0-22_5 | 1.46 | 866.6>255.3 |
| | | 866.6>329.2 |
| PC (38:5) PC 18_0-20_5 | 1.45 | 866.6>283.3 |
| | | 866.6>301.2 |
| PC 16_0-22_6 | 1.46 | 864.6>255.3 |
| | | 864.6>327.2 |
| PC 18_0-22_4 | 1.44 | 896.6>283.3 |
| | | 896.6>331.2 |
| PC 18_0-22_5 | 1.44 | 894.6>283.3 |
| | | 894.6>329.2 |

**Table 32**

| **Sphingomyelin (SM)** | **RT (mins)** | **MRM transition** |
|---|---|---|
| SM d18_1-12_0 | 2.02 | 647.5>184.1 |
| SM d18_1-14_0 | 1.99 | 675.5>184.1 |
| SM d18_1-14_1 | 2.00 | 673.5>184.1 |
| SM d18_1-16_0 | 1.96 | 703.6>184.1 |
| SM d18_1-16_1 | 1.96 | 701.6>184.1 |
| SM d18_1-17_0 | 1.94 | 717.6>184.1 |
| SM d18_1-18_0 | 1.93 | 731.6>184.1 |
| SM d18_1-18_1 | 1.93 | 729.6>184.1 |
| SM d18_1-18_2 | 1.93 | 727.6>184.1 |
| SM d18_1-18_3 | 1.93 | 725.6>184.1 |
| SM d18_1-18_4 | 1.96 | 723.5>184.1 |
| SM d18_1-20_0 | 1.91 | 759.6>184.1 |
| SM d18_1-20_1 | 1.91 | 757.6>184.1 |
| SM d18_1-20_2 | 1.90 | 755.6>184.1 |
| SM d18_1-20 3 | 1.91 | 753.6>184.1 |
| SM d18_1-20_4 | 1.90 | 751.6>184.1 |
| SM d18_1-22_0 | 1.89 | 787.7>184.1 |
| SM d18_1-22_1 | 1.89 | 785.7>184.1 |
| SM d18_1-22_2 | 1.90 | 783.6>184.1 |
| SM d18_1-22_3 | 1.90 | 781.6>184.1 |
| SM d18_1-22_4 | 1.89 | 779.6>184.1 |
| SM d18_1-22_5 | 1.89 | 777.6>184.1 |
| SM d18_1-22_6 | 1.89 | 775.6>184.1 |

As demonstrated in this example, the use of the HILIC-based quantification method discussed herein provides the advantage of performing targeted screens for LPC lipids, PC lipids, and SM lipids.

In describing example embodiments, specific terminology is used for the sake of clarity. For purposes of description, each specific term is intended to at least include all technical and functional equivalents that operate in a similar manner to accomplish a similar purpose. Additionally, in some instances where a particular example embodiment includes a plurality of system elements, device components or method steps, those elements, components or steps can be replaced with a single element, component or step. Likewise, a single element, component or step can be replaced with a plurality of elements, components or steps that serve the same purpose. Moreover, while example embodiments have been shown and described with references to particular embodiments thereof, those of ordinary skill in the art will understand that various substitutions and alterations in form and detail can be made therein without departing from the scope of the disclosure. Further still, other aspects, functions and advantages are also within the scope of the disclosure.

Example flowcharts are provided herein for illustrative purposes and are non-limiting examples of methodologies. One of ordinary skill in the art will recognize that example methodologies can include more or fewer steps than those illustrated in the example flowcharts, and that the steps in the example flowcharts can be performed in a different order than the order shown in the illustrative flowcharts.

## Claims

1. A method for identifying and quantifying lipids in a biological sample comprising classes of lipids, the method comprising:
providing a library that comprises multiple reaction monitoring (MRM) transitions for individual lipids within the classes of lipids in the biological sample;
combining known concentrations of stable isotope labeled (SIL) lipid standards with the biological sample to form a sample matrix, wherein the sample matrix includes at least one SIL lipid standard for each class of lipid in the biological sample that is being detected;
introducing the sample matrix into a liquid chromatography (LC) system to separate lipids in the sample matrix into discrete bands of lipids, wherein each discrete band of lipids comprises lipids of the same class, and wherein each class of lipids appears in a single band;
directing each of the separated discrete classes of lipids to a mass spectrometry (MS) system configured to obtain MRM transitions of the separated lipids ;
identifying lipids in the sample matrix by comparing the obtained MRM transitions to MRM transitions in the library; and
quantifying the identified lipids in each discrete class using the known concentrations of the SIL lipid standards
**characterised in that**:
the biological sample comprises lysophosphatidylcholines (LPC), phosphatidylcholines (PC), and sphingomyelins (SM);
the SIL lipid standards comprise a LPC, a PC, and a SM; and
wherein the LC system separates the lipids in the sample matrix into three discrete bands, the first discrete band comprising PCs, the second discrete band comprising SMs and the third discrete band comprising SM.

2. A method for identifying and quantifying lipids in a biological sample comprising classes of lipids, the method comprising:
providing a library that comprises multiple reaction monitoring (MRM) transitions for individual lipids within the classes of lipids in the biological sample;
combining known concentrations of stable isotope labeled (SIL) lipid standards with the biological sample to form a sample matrix, wherein the sample matrix includes at least one SIL lipid standard for each class of lipid in the biological sample that is being detected;
introducing the sample matrix into a liquid chromatography (LC) system to separate lipids in the sample matrix into discrete bands of lipids, wherein each discrete band of lipids comprises lipids of the same class, and wherein each class of lipids appears in a single band;
directing each of the separated discrete classes of lipids to a mass spectrometry (MS) system configured to obtain MRM transitions of the separated lipids ;
identifying lipids in the sample matrix by comparing the obtained MRM transitions to MRM transitions in the library; and
quantifying the identified lipids in each discrete class using the known concentrations of the SIL lipid standards
**characterised in that**:
the biological sample comprises triradylglycerolipid (TG), ceramides (CER), hexosylceramides (HexCer) and sphingomyelins (SM);
the SIL lipid standards comprise a triradylglycerolipid (TG) and a SM; and
wherein the LC system separates the lipids in the sample matrix into two discrete bands, the first discrete band comprising TGs and the second discrete band comprising CERs, HexCers, and SMs.

3. A method for identifying and quantifying lipids in a biological sample comprising classes of lipids, the method comprising:
providing a library that comprises multiple reaction monitoring (MRM) transitions for individual lipids within the classes of lipids in the biological sample;
combining known concentrations of stable isotope labeled (SIL) lipid standards with the biological sample to form a sample matrix, wherein the sample matrix includes at least one SIL lipid standard for each class of lipid in the biological sample that is being detected;
introducing the sample matrix into a liquid chromatography (LC) system to separate lipids in the sample matrix into discrete bands of lipids, wherein each discrete band of lipids comprises lipids of the same class, and wherein each class of lipids appears in a single band;
directing each of the separated discrete classes of lipids to a mass spectrometry (MS) system configured to obtain MRM transitions of the separated lipids ;
identifying lipids in the sample matrix by comparing the obtained MRM transitions to MRM transitions in the library; and
quantifying the identified lipids in each discrete class using the known concentrations of the SIL lipid standards
**characterised in that**:
the biological sample comprises monoradylglycerolipids (MG), diradylglycerolipids (DG), triradylglycerolipids (TG), lysophosphatidylcholines (LPC), lysophosphatidylethanolamines (LPE), phosphatidylcholines (PC), sphingomyelins (SM), phosphatidic acids (PA), phosphatidylethanolamines (PE), phosphatidylglycerols (PG), phosphatidylinositols (PI), cholesterol, and cholesterol ester;
the SIL lipid standards comprise a MG, a DG, a TG, a LPC, a LPE, a PC, a SM, a PA, a PE, a PG, a PI, cholesterol, and a CE; and
wherein the LC system separates the lipids in the sample matrix into eight discrete bands: the first discrete band comprising MGs, DGs, TGs, cholesterol and cholesterol ester; the second discrete band comprising PGs; the third discrete band comprising PCs; the fourth discrete band comprising PEs; the fifth discrete band comprising SMs; the sixth discrete band comprising LPCs; the seventh discrete band comprising LPEs, and the eighth discrete band comprising PIs and PAs.

4. A method for identifying and quantifying lipids in a biological sample comprising classes of lipids, the method comprising:
providing a library that comprises multiple reaction monitoring (MRM) transitions for individual lipids within the classes of lipids in the biological sample;
combining known concentrations of stable isotope labeled (SIL) lipid standards with the biological sample to form a sample matrix, wherein the sample matrix includes at least one SIL lipid standard for each class of lipid in the biological sample that is being detected;
introducing the sample matrix into a liquid chromatography (LC) system to separate lipids in the sample matrix into discrete bands of lipids, wherein each discrete band of lipids comprises lipids of the same class, and wherein each class of lipids appears in a single band;
directing each of the separated discrete classes of lipids to a mass spectrometry (MS) system configured to obtain MRM transitions of the separated lipids ;
identifying lipids in the sample matrix by comparing the obtained MRM transitions to MRM transitions in the library; and
quantifying the identified lipids in each discrete class using the known concentrations of the SIL lipid standards
**characterised in that**:
the biological sample comprises lysophosphatidylethanolamines (LPE), phosphatidylethanolamines (PE), phosphatidylglycerols (PG), and phosphatidylinositols (PI);
the SIL lipid standards comprise a LPE, a PE, a PG, and a PI; and
wherein the LC system separates the lipids in the sample matrix into four discrete bands, the first discrete band comprising PGs, the second discrete band comprising PEs, the third discrete band comprising LPEs and the fourth discrete band comprising PIs.

5. The method of any one of claims 1 to 4, wherein the LC system is a hydrophilic interaction chromatography (HILIC) system.

6. The method of any one of claims 1 to 4, wherein the MRM transitions are performed in both positive and negative ion mode.

7. The method of claim 6, wherein the MRM transitions can be fatty acyl chain fragments, head group fragments, or neutral loss fragments.

8. The method of any one of claims 1 to 4, further comprising: extracting the lipids from the sample matrix includes using protein precipitation with pre-cooled isopropanol.

9. The method of any one of claims 1 to 4, wherein the library further comprises liquid chromatography (LC) conditions for separating classes of lipids and retention times for each class of lipids present in the sample matrix, and wherein the classes of lipids are separated using the LC conditions obtained from the library.

10. The method of any one of claims 1 to 4, wherein the library further comprises mass spectrometry (MS) conditions for obtaining MRM transitions for each class of lipids, and wherein the MRM transitions for each class of lipids are generated using the MS conditions obtained from the library.

11. The method of any one of claims 1 to 4 for identifying potential biomarkers, the method comprising:
obtaining a number of biological samples associated with a medical condition, the biological samples comprising different classes of lipids;
and
determining a relationship between the identified and quantified lipids and the medical condition.

12. The method of any one of claims 1 to 4 for diagnostic screening for a medical condition associated with one or more lipids, the method comprising:
obtaining a biological sample associated with the medical condition, the biological sample comprising different classes of lipids;
and
identifying the medical condition based on the identified and quantified lipids in the biological sample.

## Patentansprüche

1. Verfahren zum Identifizieren und Quantifizieren von Lipiden in einer biologischen Probe, die Lipidklassen umfasst, das Verfahren umfassend:
Bereitstellen einer Bibliothek, die für einzelne Lipide innerhalb der Lipidklassen in der biologischen Probe Übergänge des Multiple Reaction Monitoring (MRM) umfasst;
Kombinieren bekannter Konzentrationen von mit stabilen Isotopen markierten (SIL) Lipidstandards mit der biologischen Probe zum Bilden einer Probenmatrix, wobei die Probenmatrix für jede Lipidklasse in der biologischen Probe, die detektiert wird, mindestens einen SIL-Lipidstandard enthält;
Einbringen der Probenmatrix in ein Flüssigkeitschromatographiesystem (LC-System), um Lipide in der Probenmatrix in diskrete Lipidbanden zu trennen, wobei jede diskrete Lipidbande Lipide derselben Klasse umfasst und wobei jede Lipidklasse in einer einzelnen Bande erscheint;
Leiten der einzelnen getrennten diskreten Lipidklassen zu einem Massenspektrometriesystem (MS-System), das dazu konfiguriert ist, MRM-Übergänge der getrennten Lipide zu erhalten;
Identifizieren von Lipiden in der Probenmatrix durch Vergleichen der erhaltenen MRM-Übergänge mit MRM-Übergängen in der Bibliothek; und
Quantifizieren der identifizierten Lipide in jeder diskreten Klasse unter Verwendung der bekannten Konzentrationen der SIL-Lipidstandards
**dadurch gekennzeichnet, dass**:
die biologische Probe Lysophosphatidylcholine (LPC), Phosphatidylcholine (PC) und Sphingomyeline (SM) umfasst;
die SIL-Lipidstandards ein LPC, ein PC und ein SM umfassen; und
wobei das LC-System die Lipide in der Probenmatrix in drei diskrete Banden trennt, wobei die erste diskrete Bande PCs umfasst, die zweite diskrete Bande SMs umfasst und die dritte diskrete Bande SM umfasst.

2. Verfahren zum Identifizieren und Quantifizieren von Lipiden in einer biologischen Probe, die Lipidklassen umfasst, das Verfahren umfassend:
Bereitstellen einer Bibliothek, die für einzelne Lipide innerhalb der Lipidklassen in der biologischen Probe Übergänge des Multiple Reaction Monitoring (MRM) umfasst;
Kombinieren bekannter Konzentrationen von mit stabilen Isotopen markierten (SIL) Lipidstandards mit der biologischen Probe zum Bilden einer Probenmatrix, wobei die Probenmatrix für jede Lipidklasse in der biologischen Probe, die detektiert wird, mindestens einen SIL-Lipidstandard enthält;
Einbringen der Probenmatrix in ein Flüssigkeitschromatographiesystem (LC-System), um Lipide in der Probenmatrix in diskrete Lipidbanden zu trennen, wobei jede diskrete Lipidbande Lipide derselben Klasse umfasst und wobei jede Lipidklasse in einer einzelnen Bande erscheint;
Leiten der einzelnen getrennten diskreten Lipidklassen zu einem Massenspektrometriesystem (MS-System), das dazu konfiguriert ist, MRM-Übergänge der getrennten Lipide zu erhalten;
Identifizieren von Lipiden in der Probenmatrix durch Vergleichen der erhaltenen MRM-Übergänge mit MRM-Übergängen in der Bibliothek; und
Quantifizieren der identifizierten Lipide in jeder diskreten Klasse unter Verwendung der bekannten Konzentrationen der SIL-Lipidstandards
**dadurch gekennzeichnet, dass:**
die biologische Probe Triradylglycerollipid (TG), Ceramide (CER), Hexosylceramide (HexCer) und Sphingomyeline (SM) umfasst;
die SIL-Lipidstandards ein Triradylglycerollipid (TG) und ein SM umfassen; und
wobei das LC-System die Lipide in der Probenmatrix in zwei diskrete Banden trennt, wobei die erste diskrete Bande TGs umfasst und die zweite diskrete Bande CERs, HexCers und SMs umfasst.

3. Verfahren zum Identifizieren und Quantifizieren von Lipiden in einer biologischen Probe, die Lipidklassen umfasst, das Verfahren umfassend:
Bereitstellen einer Bibliothek, die für einzelne Lipide innerhalb der Lipidklassen in der biologischen Probe Übergänge des Multiple Reaction Monitoring (MRM) umfasst;
Kombinieren bekannter Konzentrationen von mit stabilen Isotopen markierten (SIL) Lipidstandards mit der biologischen Probe zum Bilden einer Probenmatrix, wobei die Probenmatrix für jede Lipidklasse in der biologischen Probe, die detektiert wird, mindestens einen SIL-Lipidstandard enthält;
Einbringen der Probenmatrix in ein Flüssigkeitschromatographiesystem (LC-System), um Lipide in der Probenmatrix in diskrete Lipidbanden zu trennen, wobei jede diskrete Lipidbande Lipide derselben Klasse umfasst und wobei jede Lipidklasse in einer einzelnen Bande erscheint;
Leiten der einzelnen getrennten diskreten Lipidklassen zu einem Massenspektrometriesystem (MS-System), das dazu konfiguriert ist, MRM-Übergänge der getrennten Lipide zu erhalten;
Identifizieren von Lipiden in der Probenmatrix durch Vergleichen der erhaltenen MRM-Übergänge mit MRM-Übergängen in der Bibliothek; und
Quantifizieren der identifizierten Lipide in jeder diskreten Klasse unter Verwendung der bekannten Konzentrationen der SIL-Lipidstandards
**dadurch gekennzeichnet, dass:**
die biologische Probe Monoradylglycerolipide (MG), Diradylglycerolipide (DG), Triradylglycerolipide (TG), Lysophosphatidylcholine (LPC), Lysophosphatidylethanolamine (LPE), Phosphatidylcholine (PC), Sphingomyeline (SM), Phosphatidsäuren (PA), Phosphatidylethanolamine (PE), Phosphatidylglycerine (PG), Phosphatidylinositole (PI), Cholesterin und Cholesterinester umfasst;
die SIL-Lipidstandards ein MG, ein DG, ein TG, ein LPC, ein LPE, ein PC, ein SM, ein PA, ein PE, ein PG, ein Pl, Cholesterin und ein CE umfassen; und
wobei das LC-System die Lipide in der Probenmatrix in acht diskrete Banden trennt, wobei die erste diskrete Bande MGs, DGs, TGs, Cholesterin und Cholesterinester umfasst; die zweite diskrete Bande PGs umfasst; die dritte diskrete Bande PCs umfasst; die vierte diskrete Bande PEs umfasst; die fünfte diskrete Bande SMs umfasst; die sechste diskrete Bande LPCs umfasst; die siebte diskrete Bande LPEs umfasst und die achte diskrete Bande Pls und PAs umfasst.

4. Verfahren zum Identifizieren und Quantifizieren von Lipiden in einer biologischen Probe, die Lipidklassen umfasst, das Verfahren umfassend:
Bereitstellen einer Bibliothek, die für einzelne Lipide innerhalb der Lipidklassen in der biologischen Probe Übergänge des Multiple Reaction Monitoring (MRM) umfasst;
Kombinieren bekannter Konzentrationen von mit stabilen Isotopen markierten (SIL) Lipidstandards mit der biologischen Probe zum Bilden einer Probenmatrix, wobei die Probenmatrix für jede Lipidklasse in der biologischen Probe, die detektiert wird, mindestens einen SIL-Lipidstandard enthält;
Einbringen der Probenmatrix in ein Flüssigkeitschromatographiesystem (LC-System), um Lipide in der Probenmatrix in diskrete Lipidbanden zu trennen, wobei jede diskrete Lipidbande Lipide derselben Klasse umfasst und wobei jede Lipidklasse in einer einzelnen Bande erscheint;
Leiten der einzelnen getrennten diskreten Lipidklassen zu einem Massenspektrometriesystem (MS-System), das dazu konfiguriert ist, MRM-Übergänge der getrennten Lipide zu erhalten;
Identifizieren von Lipiden in der Probenmatrix durch Vergleichen der erhaltenen MRM-Übergänge mit MRM-Übergängen in der Bibliothek; und
Quantifizieren der identifizierten Lipide in jeder diskreten Klasse unter Verwendung der bekannten Konzentrationen der SIL-Lipidstandards
**dadurch gekennzeichnet, dass:**
die biologische Probe Lysophosphatidylethanolamine (LPE), Phosphatidylethanolamine (PE), Phosphatidylglycerine (PG) und Phosphatidylinositole (PI) umfasst;
die SIL-Lipidstandards ein LPE, ein PE, ein PG und ein PI umfassen; und
wobei das LC-System die Lipide in der Probenmatrix in vier diskrete Banden trennt, wobei die erste diskrete Bande PGs umfasst, die zweite diskrete Bande PEs umfasst, die dritte diskrete Bande LPEs umfasst und die vierte diskrete Bande Pls umfasst.

5. Verfahren nach einem der Ansprüche 1 bis 4, wobei das LC-System ein System zur hydrophile Interaktionschromatographie (HILIC-System) ist.

6. Verfahren nach einem der Ansprüche 1 bis 4, wobei die MRM-Übergänge sowohl im positiven als auch im negativen lonenmodus durchgeführt werden.

7. Verfahren nach Anspruch 6, wobei die MRM-Übergänge Fettsäurekettenfragmente, Kopfgruppenfragmente oder Neutralverlustfragmente sein können.

8. Verfahren nach einem der Ansprüche 1 bis 4, ferner umfassend: Das Extrahieren der Lipide aus der Probenmatrix schließt die Verwendung einer Proteinfällung mit vorgekühltem Isopropanol ein.

9. Verfahren nach einem der Ansprüche 1 bis 4, wobei die Bibliothek ferner für jede Lipidklasse, die in der Probenmatrix vorhanden ist, Flüssigkeitschromatographie-Bedingungen (LC-Bedingungen) zum Trennen von Lipidklassen und Retentionszeiten umfasst und wobei die Lipidklassen unter Verwendung der aus der Bibliothek erhaltenen LC-Bedingungen getrennt werden.

10. Verfahren nach einem der Ansprüche 1 bis 4, wobei die Bibliothek ferner für jede Lipidklasse Massenspektrometrie-Bedingungen (MS-Bedingungen) zum Erhalten von MRM-Übergängen umfasst und wobei die MRM-Übergänge für jede Lipidklasse unter Verwendung der aus der Bibliothek erhaltenen MS-Bedingungen erzeugt werden.

11. Verfahren nach einem der Ansprüche 1 bis 4 zum Identifizieren potentieller Biomarker, das Verfahren umfassend:
Erhalten einer Anzahl biologischer Proben, die mit einem medizinischen Befund im Zusammenhang stehen, wobei die biologischen Proben verschiedene Lipidklassen umfassen;
und
Bestimmen einer Beziehung zwischen den identifizierten und quantifizierten Lipiden und dem medizinischen Befund.

12. Verfahren nach einem der Ansprüche 1 bis 4 zum diagnostischen Screening auf einen medizinischen Befund, der mit einem oder mehreren Lipiden im Zusammenhang steht, das Verfahren umfassend:
Erhalten einer biologischen Probe, die mit einem medizinischen Befund im Zusammenhang steht, wobei die biologische Probe verschiedene Lipidklassen umfasst;
und
Identifizieren des medizinischen Befunds basierend auf den identifizierten und quantifizierten Lipiden in der biologischen Probe.

## Revendications

1. Procédé d'identification et de quantification de lipides dans un échantillon biologique comprenant des classes de lipides, le procédé comprenant :
la fourniture d'une bibliothèque qui comprend des transitions de surveillance de réactions multiples (MRM) pour des lipides individuels au sein des classes de lipides dans l'échantillon biologique ;
la combinaison de concentrations connues d'étalons lipidiques marqués par des isotopes stables (SIL) avec l'échantillon biologique pour former une matrice d'échantillon, dans lequel la matrice d'échantillon comporte au moins un étalon lipidique SIL pour chaque classe de lipides dans l'échantillon biologique qui est détecté ;
l'introduction de la matrice d'échantillon dans un système de chromatographie en phase liquide (CL) pour séparer des lipides dans la matrice d'échantillon en bandes distinctes de lipides, dans lequel chaque bande distincte de lipides comprend des lipides de la même classe, et dans lequel chaque classe de lipides apparaît dans une bande unique ;
la direction de chacune des classes distinctes de lipides séparées vers un système de spectrométrie de masse (MS) configuré pour obtenir des transitions MRM des lipides séparés ;
l'identification de lipides dans la matrice d'échantillon en comparant les transitions MRM obtenues aux transitions MRM dans la bibliothèque ; et
la quantification des lipides identifiés dans chaque classe distincte à l'aide des concentrations connues des étalons lipidiques SIL
**caractérisé en ce que :**
l'échantillon biologique comprend des lysophosphatidylcholines (LPC), des phosphatidylcholines (PC) et des sphingomyélines (SM) ;
les étalons lipidiques SIL comprennent une LPC, une PC et une SM ; et
dans lequel le système CL sépare les lipides dans la matrice d'échantillon en trois bandes distinctes, la première bande distincte comprenant des PC, la deuxième bande distincte comprenant des SM et la troisième bande distincte comprenant une SM.

2. Procédé d'identification et de quantification de lipides dans un échantillon biologique comprenant des classes de lipides, le procédé comprenant :
la fourniture d'une bibliothèque qui comprend des transitions de surveillance de réactions multiples (MRM) pour des lipides individuels au sein des classes de lipides dans l'échantillon biologique ;
la combinaison de concentrations connues d'étalons lipidiques marqués par des isotopes stables (SIL) avec l'échantillon biologique pour former une matrice d'échantillon, dans lequel la matrice d'échantillon comporte au moins un étalon lipidique SIL pour chaque classe de lipides dans l'échantillon biologique qui est détecté ;
l'introduction de la matrice d'échantillon dans un système de chromatographie en phase liquide (CL) pour séparer des lipides dans la matrice d'échantillon en bandes distinctes de lipides, dans lequel chaque bande distincte de lipides comprend des lipides de la même classe, et dans lequel chaque classe de lipides apparaît dans une bande unique ;
la direction de chacune des classes distinctes de lipides séparées vers un système de spectrométrie de masse (MS) configuré pour obtenir des transitions MRM des lipides séparés ;
l'identification de lipides dans la matrice d'échantillon en comparant les transitions MRM obtenues aux transitions MRM dans la bibliothèque ; et
la quantification des lipides identifiés dans chaque classe distincte à l'aide des concentrations connues des étalons lipidiques SIL
**caractérisé en ce que :**
l'échantillon biologique comprend des triradylglycérolipides (TG), des céramides (CER), des hexosylcéramides (HexCer) et des sphingomyélines (SM) ;
les étalons lipidiques SIL comprennent un triradylglycérolipide (TG) et une SM ; et
dans lequel le système CL sépare les lipides dans la matrice d'échantillon en deux bandes distinctes, la première bande distincte comprenant des TG et la seconde bande distincte comprenant des CER, des HexCers et des SM.

3. Procédé d'identification et de quantification de lipides dans un échantillon biologique comprenant des classes de lipides, le procédé comprenant :
la fourniture d'une bibliothèque qui comprend des transitions de surveillance de réactions multiples (MRM) pour des lipides individuels au sein des classes de lipides dans l'échantillon biologique ;
la combinaison des concentrations connues d'étalons lipidiques marqués par des isotopes stables (SIL) avec l'échantillon biologique pour former une matrice d'échantillon, dans lequel la matrice d'échantillon comporte au moins un étalon lipidique SIL pour chaque classe de lipides dans l'échantillon biologique qui est détecté ;
l'introduction de la matrice d'échantillon dans un système de chromatographie en phase liquide (CL) pour séparer des lipides dans la matrice d'échantillon en bandes distinctes de lipides, dans lequel chaque bande distincte de lipides comprend des lipides de la même classe, et dans lequel chaque classe de lipides apparaît dans une bande unique ;
la direction de chacune des classes distinctes de lipides séparées vers un système de spectrométrie de masse (MS) configuré pour obtenir des transitions MRM des lipides séparés ;
l'identification de lipides dans la matrice d'échantillon en comparant les transitions MRM obtenues aux transitions MRM dans la bibliothèque ; et
la quantification des lipides identifiés dans chaque classe distincte à l'aide des concentrations connues des étalons lipidiques SIL
**caractérisé en ce que :**
l'échantillon biologique comprend des monoradylglycérolipides (MG), des diradylglycérolipides (DG), des triradylglycérolipides (TG), des lysophosphatidylcholines (LPC), des lysophosphatidyléthanolamines (LPE), des phosphatidylcholines (PC), des sphingomyélines (SM), des acides phosphatidiques (PA), des phosphatidyléthanolamines (PE), des phosphatidylglycérols (PG), des phosphatidylinositols (PI), du cholestérol et de l'ester de cholestérol ;
les étalons lipidiques SIL comprennent un MG, un DG, un TG, une LPC, une LPE, une PC, une SM, un PA, une PE, un PG, un PI, un cholestérol et un CE ; et
dans lequel le système CL sépare les lipides dans la matrice d'échantillon en huit bandes distinctes : la première bande distincte comprenant des MG, des DG, des TG, du cholestérol et un ester de cholestérol ; la deuxième bande distincte comprenant des PG ; la troisième bande distincte comprenant des PC ; la quatrième bande distincte comprenant des PE ; la cinquième bande distincte comprenant des SM ; la sixième bande distincte comprenant des LPC ; la septième bande distincte comprenant des LPE, et la huitième bande distincte comprenant des PI et des PA.

4. Procédé d'identification et de quantification de lipides dans un échantillon biologique comprenant des classes de lipides, le procédé comprenant :
la fourniture d'une bibliothèque qui comprend des transitions de surveillance de réactions multiples (MRM) pour des lipides individuels au sein des classes de lipides dans l'échantillon biologique ;
la combinaison de concentrations connues d'étalons lipidiques marqués par des isotopes stables (SIL) avec l'échantillon biologique pour former une matrice d'échantillon, dans lequel la matrice d'échantillon comporte au moins un étalon lipidique SIL pour chaque classe de lipides dans l'échantillon biologique qui est détecté ;
l'introduction de la matrice d'échantillon dans un système de chromatographie en phase liquide (CL) pour séparer des lipides dans la matrice d'échantillon en bandes distinctes de lipides, dans lequel chaque bande distincte de lipides comprend des lipides de la même classe, et dans lequel chaque classe de lipides apparaît dans une bande unique ;
la direction de chacune des classes distinctes de lipides séparées vers un système de spectrométrie de masse (MS) configuré pour obtenir des transitions MRM des lipides séparés ;
l'identification de lipides dans la matrice d'échantillon en comparant les transitions MRM obtenues à des transitions MRM dans la bibliothèque ; et
la quantification des lipides identifiés dans chaque classe distincte à l'aide des concentrations connues des étalons lipidiques SIL
**caractérisé en ce que :**
l'échantillon biologique comprend des lysophosphatidyléthanolamines (LPE), des phosphatidyléthanolamines (PE), des phosphatidylglycérols (PG) et des phosphatidylinositols (PI) ;
les étalons lipidiques SIL comprennent une LPE, une PE, un PG et un PI ; et
dans lequel le système CL sépare les lipides dans la matrice d'échantillon en quatre bandes distinctes, la première bande distincte comprenant des PG, la deuxième bande distincte comprenant des PE, la troisième bande distincte comprenant des LPE et la quatrième bande distincte comprenant des PI.

5. Procédé selon l'une quelconque des revendications 1 à 4, dans lequel le système CL est un système de chromatographie d'interaction hydrophile (HILIC).

6. Procédé selon l'une quelconque des revendications 1 à 4, dans lequel les transitions MRM sont effectuées à la fois en mode d'ions positifs et négatifs.

7. Procédé selon la revendication 6, dans lequel les transitions MRM peuvent être des fragments de chaîne acyle gras, des fragments de groupe de tête ou des fragments de perte neutre.

8. Procédé selon l'une quelconque des revendications 1 à 4, comprenant en outre : l'extraction des lipides de la matrice d'échantillon comporte l'utilisation d'une précipitation de protéines avec de l'isopropanol pré-refroidi.

9. Procédé selon l'une quelconque des revendications 1 à 4, dans lequel la bibliothèque comprend en outre des conditions de chromatographie en phase liquide (CL) permettant de séparer des classes de lipides et des temps de rétention pour chaque classe de lipides présents dans la matrice d'échantillon, et dans lequel les classes de lipides sont séparées à l'aide des conditions CL obtenues à partir de la bibliothèque.

10. Procédé selon l'une quelconque des revendications 1 à 4, dans lequel la bibliothèque comprend en outre des conditions de spectrométrie de masse (MS) permettant d'obtenir des transitions MRM pour chaque classe de lipides, et dans lequel les transitions MRM pour chaque classe de lipides sont générées à l'aide des conditions MS obtenues à partir de la bibliothèque.

11. Procédé selon l'une quelconque des revendications 1 à 4 permettant d'identifier des biomarqueurs potentiels, le procédé comprenant :
l'obtention d'un nombre d'échantillons biologiques associés à une condition médicale, les échantillons biologiques comprenant différentes classes de lipides ;
et
la détermination d'une relation entre les lipides identifiés et quantifiés et la condition médicale.

12. Procédé selon l'une quelconque des revendications 1 à 4 permettant le dépistage diagnostique d'une condition médicale associée à un ou plusieurs lipides, le procédé comprenant :
l'obtention d'un échantillon biologique associé à la condition médicale l'échantillon biologique comprenant différentes classes de lipides ;
et
l'identification de la condition médicale en fonction des lipides identifiés et quantifiés dans l'échantillon biologique.
